(19) Europäisches Patentamt
European Patent Office
Office européen des brevets

(11) EP 3 679 932 A1

(12) EUROPEAN PATENT APPLICATION
published in accordance with Art. 153(4) EPC

(43) Date of publication:
**15.07.2020 Bulletin 2020/29**

(21) Application number: **18853411.9**

(22) Date of filing: **05.09.2018**

(51) Int Cl.:
***A61K 31/444*** *(2006.01)* ***A61K 31/4184*** *(2006.01)*
***A61K 31/506*** *(2006.01)* ***A61K 31/519*** *(2006.01)*
***A61K 45/00*** *(2006.01)* ***A61P 35/00*** *(2006.01)*
***A61P 35/02*** *(2006.01)* ***A61P 43/00*** *(2006.01)*

(86) International application number:
**PCT/JP2018/032880**

(87) International publication number:
**WO 2019/049891 (14.03.2019 Gazette 2019/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.09.2017 JP 2017170936**
**06.09.2017 JP 2017170950**

(71) Applicant: **ONO Pharmaceutical Co., Ltd.**
**Osaka-shi, Osaka 541-8526 (JP)**

(72) Inventors:
- **KOZAKI, Ryohei**
  **Mishima-gun**
  **Osaka 618-8585 (JP)**
- **KATO, Hikaru**
  **Mishima-gun**
  **Osaka 618-8585 (JP)**

(74) Representative: **Grünecker Patent- und Rechtsanwälte**
**PartG mbB**
**Leopoldstraße 4**
**80802 München (DE)**

(54) **METHOD FOR TREATING CANCER BY COMBINATION OF Trk INHIBITOR AND KINASE INHIBITOR**

(57) An object of the present invention is to provide a combination therapy in which an antitumor effect is enhanced as compared to when a Trk inhibitor or any of various kinase inhibitors is administered alone. A Trk inhibitor, particularly, a compound represented by General Formula (I):

[C1]

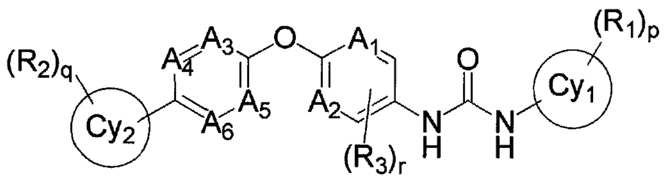

(I)

wherein all symbols have the same meanings as symbols described in this specification, a salt thereof, an N-oxide thereof, a solvate thereof, or a prodrug thereof is useful as an active ingredient of a cancer therapeutic agent used in combination with one or more drugs selected from the group consisting of an MEK inhibitor, a CDK4/6 inhibitor, an EGFR inhibitor and a JAK1/2 inhibitor.

EP 3 679 932 A1

FIG. 1

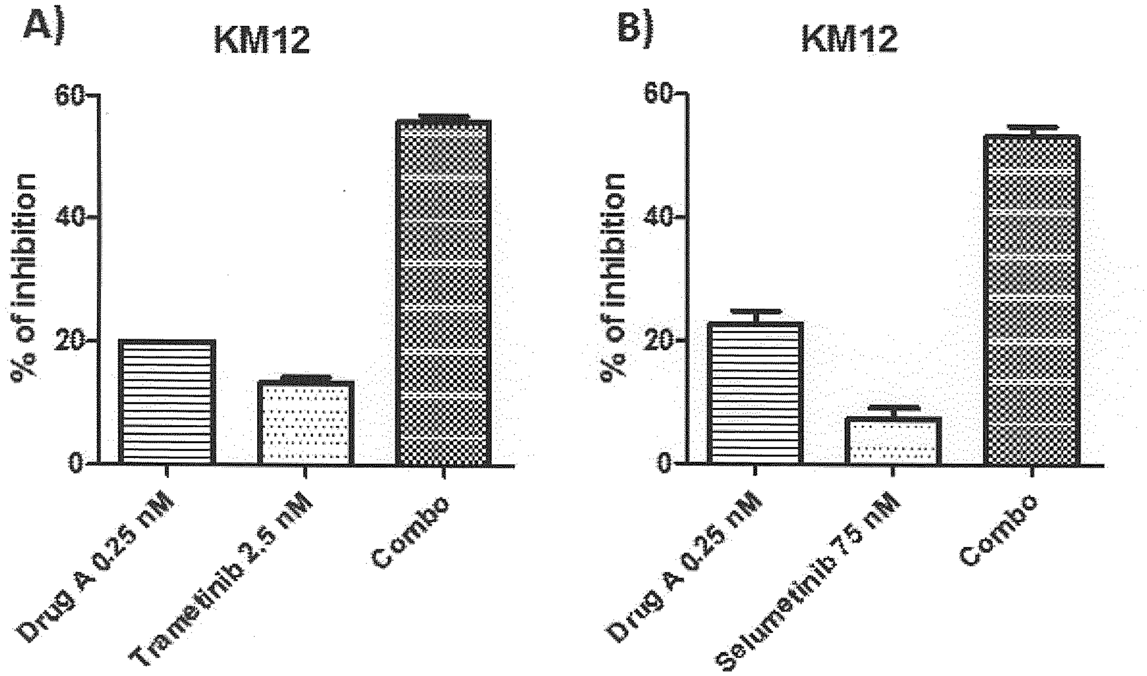
A)
KM12
% of inhibition
60
40
20
0
Drug A 0.25 nM
Trametinib 2.5 nM
Combo
B)
KM12
% of inhibition
60
40
20
0
Drug A 0.25 nM
Selumetinib 75 nM
Combo

## Description

[Technical Field]

The present invention relates to an agent for treating cancer, the agent being used such that the agent is administered in combination with other kinase inhibitors, the agent containing a compound represented by General Formula (I) :

[C1]

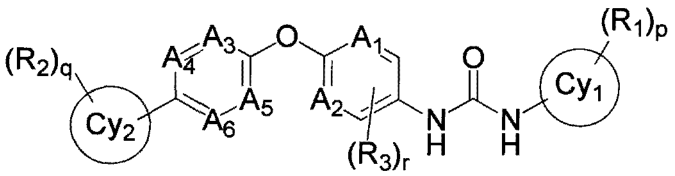

(I)

wherein all symbols have the same meanings as described hereinafter, a salt thereof, an N-oxide thereof, a solvate thereof, or a prodrug thereof, and the like.

[Background Art]

The tropomyosin receptor kinase (hereinafter abbreviated as, Trk) family belongs to receptor tyrosine kinases and is classified as TrkA which is a high-affinity receptor of a nerve growth factor (hereinafter abbreviated as NGF), TrkB which is a high-affinity receptor of a brain-derived neutrophic factor (BDNF) and neurotrophin (hereinafter abbreviated as NT)-4/5, and TrkC which is a high-affinity receptor of NT-3. TrkA, TrkB and TrkC proteins are encoded by NTRK1, NTRK2 and NTRK3 genes, respectively. All Trk receptors are highly expressed in nerve tissues and are involved in differentiation and maintenance of functions of nerve cells (refer to NPL 1). Meanwhile, it has been reported that Trk receptors are also expressed in cancer cells such as neuroblastomas, in lung cancer, breast cancer, pancreatic cancer, colon cancer, stomach cancer, liver cancer, ovarian cancer, prostate cancer, head and neck cancer, neuroendocrine tumors and blood cancer and involved in survival, proliferation, migration, and metastasis of cancer cells (NPL 2 to 8). In addition, in some of cancer patients with lung cancer, colon cancer, intrahepatic cholangiocarcinoma, thyroid cancer, skin cancer, breast cancer, head and neck cancer, renal cancer, sarcoma, brain tumor, salivary gland tumor and blood cancer, or the like, an NTRK fusion gene in which a part of the 3' side of an NTRK gene and a part of the 5' side of another gene (partner gene) are fused has been found and this fusion gene has been confirmed to present a cancer risk. Regarding the 5' side partner gene for the NTRK1 fusion gene, MPRIP, CD74, RABGAP1L, TPM3, TPR, TFG, PPL, CHTOP, ARHGEF2, NFASC, BCAN, LMNA and TP53 have been reported. Similarly, regarding the 5' side partner gene for the NTRK2 fusion gene, QKI, NACC2, VCL, AGBL4, TRIM24, PAN3, AFAP1 and SQSTM1 have been reported, and regarding the 5' side partner gene for the NTRK3 fusion gene, ETV6, BTB1, LYN and RBPMS have been reported (NPL 9 to 11). The Trk fusion protein encoded by these fusion genes is a constitutively active kinase and promotes transformation of cells into cancer cells by abnormally activating intracellular signals. Accordingly, a drug inhibiting Trk is expected to be a previously unknown novel type of anti-cancer agent.

PTL 1 describes that a compound represented by General Formula (Ie):

[C2]

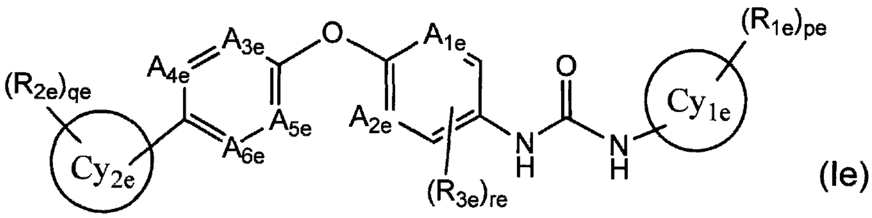

(Ie)

wherein,

a ring $Cy_{1e}$ represents a C3-10 monocyclic carbocycle or the like,

a ring $Cy_{2e}$ represents a 4- to 10-membered monocyclic heterocycle or the like,
$R_{1e}$ represents a halogen or the like,
$R_{2e}$ represents a halogen or the like,
$A_{1e}$ and $A_{2e}$ each independently represent $=CR_3-$ or the like,
$A_{3e}$, $A_{4e}$, $A_{5e}$, and $A_{6e}$ each independently represent $=CR_{4e}-$ or the like,
$R_{3e}$ and $R_{4e}$ each independently represent a hydrogen atom or the like; a part of the definitions of the groups has been excerpted), a salt thereof, an N-oxide thereof, a solvate thereof, or a prodrug thereof is a Trk inhibiting compound.

PTL 2 describes an acid addition salt of 1-{2-[4-(2-amino-5-chloro-3-pyridinyl)phenoxy]-5-pyrimidinyl}-3-[2-(methylsulfonyl)-5-(trifluoromethyl)phenyl]urea, 1-{2-[4-(2-amino-5-fluoropyridin-3-yl)phenoxylpyrimidin-5-yl}-3-[2-(pyridin-3-yl)-5-(trifluoromethyl)phenyl]urea, or 1-(2-(1H-pyrazol-1-yl)-5-(trifluoromethyl)phenyl)-3-(2-(4-(2-amino-5-chloropyridin-3-yl)phenoxy)pyrimidin-5-yl)urea which is a Trk inhibiting compound or a crystal thereof.
It has been reported in PTL 3 that, in TPM3-TrkA-G595R expressing cells, when entrectinib having Trk inhibiting activity and trametinib having MEK inhibiting activity are used in combination, proliferation of cells is inhibited.

[Citation List]

[Patent Literature]

[PTL 1] WO 2014/129431
[PTL 2] WO 2016/027754
[PTL 3] WO 2017/106492

[Non Patent Literature]

[NPL 1] Annual Review of Biochemistry, vol. 72, pp. 609-642, 2003
[NPL 2] Cancer Letters, vol. 228, pp. 143-153, 2005
[NPL 3] Clinical Cancer Research, vol. 15, pp. 5962-5967, 2009
[NPL 4] Proceedings of the National Academy of Sciences, vol. 111, pp. 10299-10304, 2014
[NPL 5] Clinical Cancer Research, vol. 7, pp. 105-112, 2001
[NPL 6] Carcinogenesis, vol. 31, pp. 1939-1947, 2010
[NPL 7] Lung Cancer, vol. 79, pp. 205-214, 2013
[NPL 8] Biochemical and Biophysical Research Communications, vol. 441, pp. 431-437, 2013
[NPL 9] Nature Medicine, vol. 19, pp. 1469-1474, 2013
[NPL 10] Cancer Discovery, vol. 5, pp. 25-34, 2015
[NPL 11] Onco gene, vol. 32, pp. 3698-3710, 2013

[Summary of Invention]

[Technical Problem]

An object of the present invention is to provide a combination therapy in which an antitumor effect is enhanced.

[Solution to Problem]

The inventors conducted extensive studies in order to achieve the above object, and as a result, found that a combination of a Trk inhibitor, specifically, a compound represented by the following General Formula (I), a salt thereof, an N-oxide thereof, a solvate thereof, or a prodrug thereof (hereinafter sometimes referred to as the compound of the present invention), and one or more drugs selected from the group consisting of an MEK inhibitor, a CDK4/6 inhibitor, an EGFR inhibitor and a JAK1/2 inhibitor (hereinafter sometimes referred to as the combination of the present invention) enhances an antitumor effect.
One embodiment of the present invention is as follows.

[1] An agent for treating cancer, the agent being used such that the agent is administered in combination with one

or more drugs selected from the group consisting of an MEK inhibitor, a CDK4/6 inhibitor, an EGFR inhibitor and a JAK1/2 inhibitor, the agent containing a compound represented by General Formula (I):

[C3]

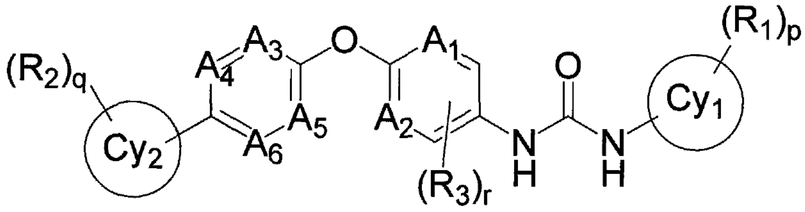

(I)

wherein,

a ring $Cy_1$ represents a C3-10 monocyclic carbocycle or bicyclic carbocycle, or a 4- to 10-membered monocyclic heterocycle or bicyclic heterocycle,
a ring $Cy_2$ represents a 4- to 10-membered monocyclic heterocycle or bicyclic heterocycle,
$R_1$ represents

(1) a halogen,
(2) a C1-6 alkyl group, C2-6 alkenyl group or C2-6 alkynyl group optionally substituted with a substituent selected from the group consisting of (i) a halogen and (ii) a hydroxy group,
(3) a C5-6 monocyclic carbocycle optionally substituted with one or two $R_5$ groups,
(4) a 5- to 6-membered monocyclic heterocycle optionally substituted with one or two $R_5$ groups,
(5) $-S(O)m_1-R_6$,
(6) $-SO_2NR_7R_8$,
(7) $-C(O)OR_9$,
(8) $-NR_{10}C(O)R_{11}$,
(9) $-C(O)NR_{12}R_{13}$,
(10) $-OR_{14}$,
(11) $-NR_{15}R_{16}$,
(12) a cyano group, or
(13) a nitro group,

$R_5$ represents

(1) a halogen,
(2) $-S(O)_{m2}-R_{17}$,
(3) $-SO_2NR_{18}R_{19}$,
(4) $-C(O)OR_{20}$,
(5) $-NR_{21}C(O)R_{22}$,
(6) $-C(O)NR_{23}R_{24}$,
(7) $-OR_{25}$,
(8) $-NR_{26}R_{27}$,
(9) a cyano group,
(10) a nitro group, or
(11) a C1-3 alkyl group optionally substituted with a substituent selected from the group consisting of (i) a halogen, (ii) a hydroxy group and (iii) an oxo group,

when two $R_5$ groups are present, the $R_5$ groups may be independently the same or different,
when, further, two $R_5$ groups are each independently a C1-3 alkyl group or a hydroxy group and the $R_5$ groups are attached to carbon atoms adjacent to each other on the C5-6 monocyclic carbocycle or the 5- to 6-membered monocyclic heterocycle, the $R_5$ groups may together form a ring,
$R_6$ to $R_{27}$ each independently represent (1) a hydrogen atom or (2) a C1-6 alkyl group optionally substituted with (i) a halogen or (ii) a hydroxy group,
when $R_{18}$ and $R_{19}$ are each independently a C1-6 alkyl group, $R_{18}$ and $R_{19}$ groups may together form a ring,
$R_2$ represents

(1) a halogen,
(2) a C1-6 alkyl group optionally substituted with (i) a halogen or (ii) a hydroxy group,
(3) a C3-6 cycloalkyl group optionally substituted with (i) a halogen or (ii) a hydroxy group,
(4) a C1-6 alkoxy group optionally substituted with a halogen,
(5) $-NR_{28}R_{29}$,
(6) a 3- to 7-membered monocyclic heterocycle, or
(7) -O-(3- to 7-membered monocyclic heterocycle),

$R_{28}$ and $R_{29}$ each independently represent (1) a hydrogen atom or (2) a Cl-6 alkyl group optionally substituted with (i) a halogen or (ii) a hydroxy group,
$A_1$ and $A_2$ each independently represent $=CR_3$- or =N-,
$A_3$, $A_4$, $A_5$ and $A_6$ each independently represent $=CR_4$- or =N-,
$R_3$ and $R_4$ each independently represent a hydrogen atom or a halogen,
m1 represents an integer of 0 to 2,
m2 represents an integer of 0 to 2,
p represents an integer of 0 to 7,
q represents an integer of 0 to 7,
r represents an integer of 0 to 2,
provided that, when $R_1$, $R_2$, $R_3$ and $R_4$ each independently occur twice or more, $R_1$, $R_2$, $R_3$ and $R_4$ may each independently be the same or different,
a salt thereof, an N-oxide thereof, a solvate thereof, or a prodrug thereof.

[2] An agent for treating cancer, the agent being used such that the agent is administered in combination with a compound represented by General Formula (I):

[C4]

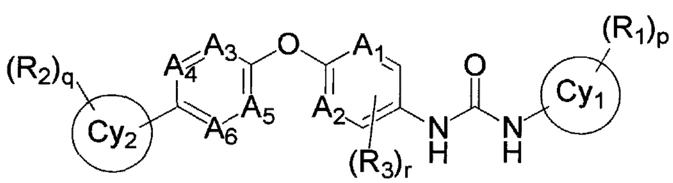

(I)

wherein all symbols have the same meanings as in [1], a salt thereof, an N-oxide thereof, a solvate thereof, or a prodrug thereof, the agent containing one or more drugs selected from the group consisting of an MEK inhibitor, a CDK4/6 inhibitor, an EGFR inhibitor and a JAK1/2 inhibitor.
[3] An agent for treating cancer, the agent containing:

one or more drugs selected from the group consisting of an MEK inhibitor, a CDK4/6 inhibitor, an EGFR inhibitor and a JAK1/2 inhibitor; and
a compound represented by General Formula (I):

[C5]

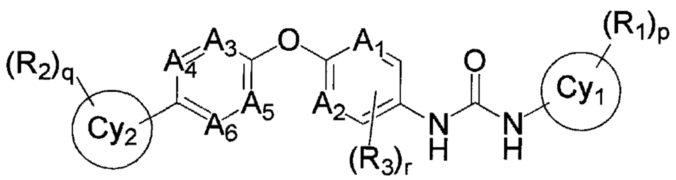

(I)

wherein all symbols have the same meanings as in [1], a salt thereof, an N-oxide thereof, a solvate thereof, or a prodrug thereof.

[4] The agent according to any one of [1] to [3],
wherein the compound represented by General Formula (I), a salt thereof, an N-oxide thereof, a solvate thereof, or a prodrug thereof is

(1) 1-(2-(1H-pyrazol-1-yl)-5-(trifluoromethyl)phenyl)-3-(2-(4-(2-amino-5-chloropyridin-3-yl)phenoxy)pyrimidin-5-yl)urea,
(2) 1-(2-(4-(2-amino-5-chloropyridin-3-yl)phenoxy)pyrimidin-5-yl)-3-(2-(4-methyl-1H-1,2,3-triazol-1-yl)-5-(trifluoromethyl)phenyl)urea,
(3) 1-(2-(4-(2-amino-5-chloropyridin-3-yl)phenoxy)pyrimidin-5-yl)-3-(5-(trifluoromethyl)-2-(3-(trifluoromethyl)-1H-pyrazol-1-yl)phenyl)urea,
(4) 1-(2-(4-(2-amino-5-chloropyridin-3-yl)phenoxy)pyrimidin-5-yl)-3-(2-chloro-5-(trifluoromethyl)phenyl)urea,
(5) 1-(2-(1H-pyrazol-1-yl)-5-(trifluoromethyl)phenyl-3-(6-(4-(2-amino-5-chloropyridin-3-yl)phenoxy)pyridin-3-yl)urea,
(6) 1-(2-(1H-1,2,3-triazol-1-yl)-5-(trifluoromethyl)phenyl-3-(6-(4-(2-amino-5-chloropyridin-3-yl)phenoxy)pyridin-3-yl)urea,
(7) 1-(6-(4-(2-amino-5-chloropyridin-3-yl)phenoxy)pyridin-3-yl)-3-(2-(pyridin-3-yl)-5-(trifluoromethyl)phenyl)urea,
(8) 1-(2-(4-(2-amino-5-chloropyridin-3-yl)phenoxy)pyrimidin-5-yl)-3-(2-(1-methyl-1H-pyrazol-5-yl)-5-(trifluoromethyl)phenyl)urea,
(9) 1-(2-(1H-1,2,3-triazol-1-yl)-5-(trifluoromethyl)phenyl)-3-(2-(4-(2-amino-5-fluoropyridin-3-yl)phenoxy)pyrimidin-5-yl)urea,
(10) 1-(2-(4-(2-amino-5-fluoropyridin-3-yl)phenoxy)pyrimidin-5-yl)-3-(2-(pyridin-3-yl)-5-(trifluoromethyl)phenyl)urea,
(11) 1-(2-(1H-pyrazol-1-yl)-4-(trifluoromethyl)phenyl)-3-(2-(4-(2-amino-5-chloropyridin-3-yl)phenoxy)pyrimidin-5-yl)urea,
(12) 1-(2-(4-(2-amino-5-chloropyridin-3-yl)phenoxy)pyrimidin-5-yl)-3-(2-fluoro-4-(trifluoromethyl)phenyl)urea,
(13) 1-(2-(4-(2-amino-5-chloropyridin-3-yl)phenoxy)pyrimidin-5-yl)-3-(2-chloro-4-(trifluoromethyl)phenyl)urea,
(14) 1-(2-{4-[2-amino-5-(trifluoromethyl)-3-pyridinyl]phenoxy}-5-pyrimidinyl)-3-{5-(trifluoromethyl)-2-[3-(trifluoromethyl)-1H-pyrazol-1-yl]phenyl}urea,
(15) 1-{2-[4-(2-amino-5-chloro-3-pyridinyl)phenoxy]-5-pyrimidinyl}-3-[4-(trifluoromethyl)-2-biphenylyl]urea,
(16) 1-{2-[4-(2-amino-5-fluoro-3-pyridinyl)phenoxy]-5-pyrimidinyl}-3-[4-(trifluoromethyl)-2-biphenylyl]urea,
(17) 1-{2-[4-(2-amino-5-chloro-3-pyridinyl)phenoxy]-5-pyrimidinyl}-3-[2-(4-chloro-1H-pyrazol-1-yl)-5-(trifluoromethyl)phenyl]urea,
(18) 1-{2-[4-(2-amino-5-chloro-3-pyridinyl)phenoxy]-5-pyrimidinyl}-3-{5-chloro-2-[3-(trifluoromethyl)-1H-pyrazol-1-yl]phenyl}urea,
(19) 1-{2-[4-(2-amino-5-chloro-3-pyridinyl)phenoxy]-5-pyrimidinyl}-3-[2,4-bis(trifluoromethyl)phenyl]urea,
(20) 1-(2-{4-[2-amino-5-(trifluoromethyl)-3-pyridinyl]phenoxy}-5-pyrimidinyl)-3-[2-(4-chloro-1H-pyrazol-1-yl)-5-(trifluoromethyl)phenyl]urea,
(21) 1-{2-[4-(2-amino-5-fluoro-3-pyridinyl)phenoxy]-5-pyrimidinyl}-3-[2-(methylsulfonyl)-5-(trifluoromethyl)phenyl]urea,
(22) 1-(2-{4-[2-amino-5-(trifluoromethyl)-3-pyridinyl]phenoxy}-5-pyrimidinyl)-3-[2-(methylsulfonyl)-5-(trifluoromethyl)phenyl]urea,
(23) 1-{2-[4-(2-amino-5-chloro-3-pyridinyl)phenoxy]-5-pyrimidinyl}-3-[2-(methylsulfonyl)-5-(trifluoromethyl)phenyl]urea,
(24) 2-{[(2-{4-[2-amino-5-(trifluoromethyl)-3-pyridinyl]phenoxy}-5-pyrimidinyl)carbamoyl] amino}-N,N-dimethyl-4-(trifluoromethyl)benzenesulfonamide,
(25) 1-(2-(4-(5-(azetidin-1-yl)pyrazolo[1,5-a]pyrimidin-3-yl)phenoxy)pyrimidin-5-yl)-3-(2-(pyridin-3-yl)-5-(trifluoromethyl)phenyl)urea,
(26) 1-(2-(4-(5-(azetidin-1-yl)pyrazolo[1,5-a]pyrimidin-3-yl)phenoxy)pyrimidin-5-yl)-3-(2-(1-methyl-1H-pyrazol-5-yl)-5-(trifluoromethyl)phenyl)urea,
(27) 1-(2-(4-(5-methoxypyrazolo[1,5-a]pyrimidin-3-yl)phenoxy)pyrimidin-5-yl)-3-(2-(pyridin-3-yl)-5-(trifluoromethyl)phenyl)urea,
(28) 1-(2-(4-(pyrazolo[1,5-a]pyrimidin-3-yl)phenoxy)pyrimidin-5-yl)-3-(2-(pyridin-3-yl)-5-(trifluoromethyl)phenyl)urea,
(29) 1-(2-{4-[5-(dimethylamino)pyrazolo[1,5-a]pyrimidin-3-yl]phenoxy}-5-pyrimidinyl)-3-[2-(3-pyridinyl)-5-(trifluoromethyl)phenyl]urea,
(30) 1-(2-{4-[5-(dimethylamino)pyrazolo[1,5-a]pyrimidin-3-yl]phenoxy}-5-pyrimidinyl)-3-[2-(1-methyl-1H-pyrazol-5-yl)-5-(trifluoromethyl)phenyl]urea,
(31) 1-{2-[4-(5-methylpyrazolo[1,5-a]pyrimidin-3-yl)phenoxy]-5-pyrimidinyl}-3-[2-(3-pyridinyl)-5-(trifluoromethyl)phenyl]urea,
(32) 1-(2-{4-[5-(ethylamino)pyrazolo[1,5-a]pyrimidin-3-yl]phenoxy}-5-pyrimidinyl)-3-[3'-methyl-4-(trifluoromethyl)-2-biphenylyl]urea,

(33) 1-(2-{4-[5-(dimethylamino)pyrazolo[1,5-a]pyrimidin-3-yl]phenoxy}-5-pyrimidinyl)-3-[4-(trifluoromethyl)-2-biphenylyl]urea,
(34) 1-(2-{4-[5-(dimethylamino)pyrazolo[1,5-a]pyrimidin-3-yl]phenoxyl-5-pyrimidinyl)-3-[3'-methyl-4-(trifluoromethyl)-2-biphenylyl]urea, or
(35) 1-(2-{4-[5-(dimethylamino)pyrazolo[1,5-a]pyrimidin-3-yl]phenoxy}-5-pyrimidinyl)-3-[2'-methyl-4-(trifluoromethyl)-2-biphenylyl]urea, a salt thereof, an N-oxide thereof, a solvate thereof, or a prodrug thereof.

[5] The agent according to any one of [1] to [4],
wherein the MEK inhibitor is trametinib or selumetinib.
[6] The agent according to any one of [1] to [4],
wherein the CDK4/6 inhibitor is palbociclib.
[7] The agent according to any one of [1] to [4],
wherein the EGFR inhibitor is one or more drugs selected from the group consisting of gefitinib, erlotinib, neratinib, canertinib and afatinib.
[8] The agent according to any one of [1] to [4],
wherein the JAK1/2 inhibitor is ruxolitinib.
[9] The agent according to any one of [1] to [8],
wherein the cancer is NTRK gene positive cancer.
[10] The agent according to [9],
wherein the NTRK gene positive cancer is NTRK fusion gene positive cancer.
[11] The agent according to any one of [1] to [10],
wherein the cancer is Trk inhibitor-resistant cancer.
[12] The agent according to [11],
wherein the Trk inhibitor-resistant cancer is cancer resistant to 1-{2-[4-(2-amino-5-chloro-3-pyridinyl)phenoxy]-5-pyrimidinyl}-3-[2-(methylsulfonyl)-5-(trifluoromethyl)phenyl]urea, a salt thereof, an N-oxide thereof, a solvate thereof, or a prodrug thereof.
[13] The agent according to [11],
wherein the Trk inhibitor-resistant cancer is cancer resistant to one or more drugs selected from the group consisting of entrectinib, LOXO-101, LOXO-195, AZD-7451, TSR-011, crizotinib, altiratinib, ASP-7269, DS-6051b and VM-902.
[14] The agent according to any one of [1] to [13],
wherein the cancer is lung cancer, colon cancer, intrahepatic cholangiocarcinoma, thyroid cancer, skin cancer, breast cancer, head and neck cancer, renal cancer, sarcoma, brain tumor, salivary gland tumor or blood cancer.
[15] A cancer treatment method including
administering an effective amount of the compound represented by General Formula (I), a salt thereof, an N-oxide thereof, a solvate thereof or a prodrug thereof according to [1] and an effective amount of one or more drugs selected from the group consisting of an MEK inhibitor, a CDK4/6 inhibitor, an EGFR inhibitor and a JAK1/2 inhibitor to a patient in need of cancer treatment simultaneously or separately.
[16] A cancer treatment method including:

administering an effective amount of the compound represented by General Formula (I), a salt thereof, an N-oxide thereof, a solvate thereof or a prodrug thereof according to [1] to a patient,
wherein the treatment further includes administering an effective amount of one or more drugs selected from the group consisting of an MEK inhibitor, a CDK4/6 inhibitor, an EGFR inhibitor and a JAK1/2 inhibitor.

[17] The compound represented by General Formula (I), a salt thereof, an N-oxide thereof, a solvate thereof or a prodrug thereof according to [1] which is used to treat cancer in combination with one or more drugs selected from the group consisting of an MEK inhibitor, a CDK4/6 inhibitor, an EGFR inhibitor and a JAK1/2 inhibitor.

[17-1] The compound represented by General Formula (I), a salt thereof, an N-oxide thereof, a solvate thereof or a prodrug thereof according to [1] which is used to treat cancer in combination with an MEK inhibitor.
[17-2] The compound represented by General Formula (I), a salt thereof, an N-oxide thereof, a solvate thereof or a prodrug thereof according to [1] which is used to treat cancer in combination with a CDK4/6 inhibitor.
[17-3] The compound represented by General Formula (I), a salt thereof, an N-oxide thereof, a solvate thereof or a prodrug thereof according to [1] which is used to treat cancer in combination with an EGFR inhibitor.
[17-4] The compound represented by General Formula (I), a salt thereof, an N-oxide thereof, a solvate thereof or a prodrug thereof according to [1] which is used to treat cancer in combination with a JAK1/2 inhibitor.

[18] An MEK inhibitor, a CDK4/6 inhibitor, an EGFR inhibitor or a JAK1/2 inhibitor which is used to treat cancer in

combination with the compound represented by General Formula (I), a salt thereof, an N-oxide thereof, a solvate thereof or a prodrug thereof according to [1].

[18-1] An MEK inhibitor which is used to treat cancer in combination with the compound represented by General Formula (I), a salt thereof, an N-oxide thereof, a solvate thereof or a prodrug thereof according to [1].
[18-2] A CDK4/6 inhibitor which is used to treat cancer in combination with the compound represented by General Formula (I), a salt thereof, an N-oxide thereof, a solvate thereof or a prodrug thereof according to [1].
[18-3] An EGFR inhibitor which is used to treat cancer in combination with the compound represented by General Formula (I), a salt thereof, an N-oxide thereof, a solvate thereof or a prodrug thereof according to [1].
[18-4] A JAK1/2 inhibitor which is used to treat cancer in combination with the compound represented by General Formula (I), a salt thereof, an N-oxide thereof, a solvate thereof or a prodrug thereof according to [1].

[19] Use of the compound represented by General Formula (I), a salt thereof, an N-oxide thereof, a solvate thereof or a prodrug thereof according to [1] for producing a cancer therapeutic agent administered in combination with one or more drugs selected from the group consisting of an MEK inhibitor, a CDK4/6 inhibitor, an EGFR inhibitor and a JAK1/2 inhibitor.
[20] An agent for treating cancer, the agent being used such that the agent is administered in combination with one or more drugs selected from the group consisting of an MEK inhibitor, a CDK4/6 inhibitor, an EGFR inhibitor and a JAK1/2 inhibitor simultaneously or separately in order to treat cancer, the agent containing the compound represented by General Formula (I), a salt thereof, an N-oxide thereof, a solvate thereof or a prodrug thereof according to [1].
[21] A pharmaceutical composition for treating cancer, including:

the compound represented by General Formula (I), a salt thereof, an N-oxide thereof, a solvate thereof or a prodrug thereof according to [1]; and
one or more drugs selected from the group consisting of an MEK inhibitor, a CDK4/6 inhibitor, an EGFR inhibitor and a JAK1/2 inhibitor.

[Advantageous Effect of Invention]

The combination of the present invention is beneficial to treat various cancers.

[Brief Description of Drawings]

[Fig. 1]
Fig. 1A) shows a growth inhibition rate with treatment with 0.25 nM of a compound A alone (left), with treatment with 2.5 nM of trametinib alone (center), and with treatment with 0.25 nM of the compound A and 2.5 nM of trametinib in combination (right), with respect to KM12 cell lines, and Fig. 1B) shows a growth inhibition rate with treatment with 0.25 nM of the compound A alone (left), with treatment with 75 nM of selumetinib alone (center), and with treatment with 0.25 nM of the compound A and 75 nM of selumetinib in combination (right), with respect to KM12 cell lines.
[Fig. 2]
Fig. 2A) shows a growth inhibition rate with treatment with 0.25 nM of the compound A alone (left), with treatment with 5 nM of trametinib alone (center), and with treatment with 0.25 nM of the compound A and 5 nM of trametinib in combination (right), with respect to IMS-M2 cell lines, and Fig. 2B) shows a growth inhibition rate with treatment with 0.25 nM of the compound A alone (left), with treatment with 300 nM of selumetinib alone (center), and with treatment with 0.25 nM of the compound A and 300 nM of selumetinib in combination (right), with respect to IMS-M2 cell lines.
[Fig. 3]
Fig. 3A) shows a growth inhibition rate with treatment with 10 nM of the compound A alone (left), with treatment with treatment with 10 nM of trametinib alone (center), and with treatment with 10 nM of the compound A and 10 nM of trametinib in combination (right), with respect to KM12-AR cell lines, and Fig. 3B) shows a growth inhibition rate with treatment with 10 nM of the compound A alone (left), with treatment with 300 nM of selumetinib alone (center), and with treatment with 10 nM of the compound A and 300 nM of selumetinib in combination (right), with respect to KM12-AR cell lines.
[Fig. 4]
Fig. 4A) shows a growth inhibition rate with treatment with 0.25 nM of the compound A alone (left), with treatment

with 30 nM of palbociclib alone (center), and with treatment with 0.25 nM of the compound A and 30 nM of palbociclib in combination (right), with respect to KM12 cell lines, Fig. 4B) shows a growth inhibition rate with treatment with 0.5 nM of the compound A alone (left), with treatment with 100 nM of afatinib alone (center), and with treatment with 0.5 nM of the compound A and 100 nM of afatinib in combination (right), with respect to KM12 cell lines, and Fig. 4C) shows a growth inhibition rate with treatment with 0.25 nM of the compound A alone (left), with treatment with 1,000 nM of erlotinib alone (center), and with treatment with 0.25 nM of the compound A and 1,000 nM of erlotinib in combination (right), with respect to KM12 cell lines.

[Fig. 5]

Fig. 5A) shows a growth inhibition rate with treatment with 0.25 nM of the compound A alone (left), with treatment with 30 nM of palbociclib alone (center), and with treatment with 0.25 nM of the compound A and 30 nM of palbociclib in combination (right), with respect to IMS-M2 cell lines, Fig. 5B) shows a growth inhibition rate with treatment with 0.25 nM of the compound A alone (left), with treatment with 300 nM of afatinib alone (center), and with treatment with 0.25 nM of the compound A and 300 nM of afatinib in combination (right), with respect to IMS-M2 cell lines, and Fig. 5C) shows a growth inhibition rate with treatment with 0.25 nM of the compound A alone (left), with treatment with 250 nM of ruxolitinib alone (center), and with treatment with 0.25 nM of the compound A and 250 nM of ruxolitinib in combination (right), with respect to IMS-M2 cell lines.

[Fig. 6]

Fig. 6A) shows a growth inhibition rate with treatment with 10 nM of the compound A alone (left), with treatment with 1,000 nM of palbociclib alone (center), and with treatment with 10 nM of the compound A and 1,000 nM of palbociclib in combination (right), with respect to KM12-AR cell lines, Fig. 6B) shows a growth inhibition rate with treatment with 10 nM of the compound A alone (left), with treatment with 30 nM of afatinib alone (center), and with treatment with 10 nM of the compound A and 30 nM of afatinib in combination (right), with respect to KM12-AR cell lines, Fig. 6C) shows a growth inhibition rate with treatment with 10 nM of the compound A alone (left), with treatment with 300 nM of erlotinib alone (center), and with treatment with 10 nM of the compound A and 300 nM of erlotinib in combination (right), with respect to KM12-AR cell lines, and Fig. 6D) shows a growth inhibition rate with treatment with 10 nM of the compound A alone (left), with treatment with 1,000 nM of gefitinib alone (center), and with treatment with 10 nM of the compound A and 1,000 nM of gefitinib in combination (right), with respect to KM12-AR cell lines.

[Fig. 7]

Fig. 7A) shows a growth inhibition rate with treatment with 10 nM of the compound A alone (left), with treatment with 300 nM of neratinib alone (center), and with treatment with 10 nM of the compound A and 300 nM of neratinib in combination (right), with respect to KM12-AR cell lines, Fig. 7B) shows a growth inhibition rate with treatment with 10 nM of the compound A alone (left), with treatment with 3,000 nM of lapatinib alone (center), and with treatment with 10 nM of the compound A and 3,000 nM of lapatinib in combination (right), with respect to KM12-AR cell lines, and Fig. 7C) shows a growth inhibition rate with treatment with 10 nM of the compound A alone (left), with treatment with 300 nM of canernib alone (center), and with treatment with 10 nM of the compound A and 300 nM of canertinib in combination (right), with respect to KM12-AR cell lines.

[Description of Embodiments]

Hereinafter, the present invention will be described in detail.

In the present invention, in one aspect, a Trk inhibitor is a compound represented by General Formula (I):

[C6]

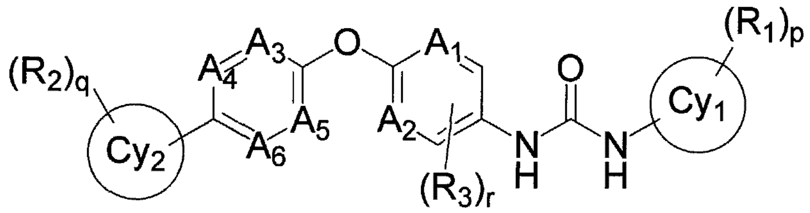

(I)

wherein,

a ring $Cy_1$ represents a C3-10 monocyclic carbocycle or bicyclic carbocycle, or a 4- to 10-membered monocyclic heterocycle or bicyclic heterocycle,

a ring $Cy_2$ represents a 4- to 10-membered monocyclic heterocycle or bicyclic heterocycle,

$R_1$ represents

(1) a halogen,
(2) a C1-6 alkyl group, C2-6 alkenyl group, or C2-6 alkynyl group which is optionally substituted with a substituent selected from the group consisting of (i) a halogen and (ii) a hydroxyl group,
(3) a C5-6 monocyclic carbocycle which is optionally substituted with one or two $R_5$ groups,
(4) a 5- to 6-membered monocyclic heterocycle which is optionally substituted with one or two $R_5$ groups,
(5) $-S(O)_{m1}-R_6$,
(6) $-SO_2NR_7R_8$,
(7) $-C(O)OR_9$,
(8) $-NR_{10}C(O)R_{11}$,
(9) $-C(O)NR_{12}R_{13}$,
(10) $-OR_{14}$,
(11) $-NR_{15}R_{16}$,
(12) a cyano group, or
(13) a nitro group,

$R_5$ represents

(1) a halogen,
(2) $-S(O)_{m2}-R_{17}$,
(3) $-SO_2NR_{18}R_{19}$,
(4) $-C(O)OR_{20}$,
(5) $-NR_{21}C(O)R_{22}$,
(6) $-C(O)NR_{23}R_{24}$,
(7) $-OR_{25}$,
(8) $-NR_{26}R_{27}$,
(9) a cyano group,
(10) a nitro group or
(11) a C1-3 alkyl group which is optionally substituted with a substituent selected from the group consisting of (i) a halogen, (ii)a hydroxyl group, and (iii) an oxo group,

when there are two $R_5$ groups, the $R_5$ groups may each independently be the same or different,
additionally, when two $R_5$ groups each independently represent a C1-3 alkyl group or a hydroxyl group and the $R_5$ groups are positioned on carbon atoms adjacent to each other on the C5-6 monocyclic carbocycle or the 5- to 6-membered monocyclic heterocycle, these groups may together form a ring,
$R_6$ to $R_{27}$ each independently represent (1) a hydrogen atom or (2) a C1-6 alkyl group which is optionally substituted with (i) a halogen or (ii) a hydroxyl group, and
when $R_{18}$ and $R_{19}$ each independently represent a C1-6 alkyl group, these groups may together form a ring,
$R_2$ represents

(1) a halogen,
(2) a C1-6 alkyl group which is optionally substituted with (i) a halogen or (ii) a hydroxyl group,
(3) a C3-6 cycloalkyl group which is optionally substituted with (i) a halogen or (ii) a hydroxyl group,
(4) a C1-6 alkoxy group which is optionally substituted with a halogen,
(5) $-NR_{28}R_{29}$,
(6) a 3- to 7-membered monocyclic heterocycle, or
(7) -O-(3- to 7-membered monocyclic heterocycle),

$R_{28}$ and $R_{29}$ each independently represent (1) a hydrogen atom, or (2) a C1-6 alkyl group which is optionally substituted with (i) a halogen or (ii) a hydroxyl group,
$A_1$ and $A_2$ each independently represent $=CR_3-$ or =N-,
$A_3$, $A_4$, $A_5$, and $A_6$ each independently represent $=CR_4-$ or =N-,
$R_3$ and $R_4$ each independently represent a hydrogen atom or a halogen,
m1 represents an integer of 0 to 2,
m2 represents an integer of 0 to 2,
p represents an integer of 0 to 7,
q represents an integer of 0 to 7,
r represents an integer of 0 to 2,
provided that, when $R_1$, $R_2$, $R_3$ and $R_4$ each independently occur twice or more, $R_1$, $R_2$, $R_3$ and $R_4$ may each

independently be the same or different,
a salt thereof, an N-oxide thereof, a solvate thereof, or a prodrug thereof.

In the present invention, "a C3-10 monocyclic carbocycle or bicyclic carbocycle" may include, for example, cyclopropane, cyclobutane, cyclopentane, cyclohexane, cycloheptane, cyclooctane, cyclononane, cyclodecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indane, naphthalene, dihydronaphthalene, tetrahydronaphthalene and perhydronaphthalene rings.

In the present invention, "a 4- to 10-membered monocyclic heterocycle or bicyclic heterocycle" may include, for example, oxetane, azetidine, pyrrolidine, pyrrole, imidazole, triazole, tetrazole, pyrazole, pyridine, piperidine, piperazine, pyrazine, pyrimidine, pyridazine, azepine, diazepine, furan, pyran, oxepin, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, indazole, quinoline, isoquinoline, quinolidine, purine, phthalazine, pteridine, naphthyridine, quinoxaline, quinazoline, cinnoline, benzoxazole, benzothiazole, benzimidazole, benzodioxole, benzoxathiole, chromene, benzofurazan, benzothiadiazole, benzotriazole, pyrroline, pyrrolidine, imidazoline, imidazolidine, triazoline, triazolidine, tetrazoline, tetrazolidine, pyrazoline, pyrazolidine, dihydropyridine, tetrahydropyridine, dihydropyrazine, tetrahydropyrazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrofuran, tetrahydrofuran, dihydropyran, tetrahydropyran, dihydrooxepin, tetrahydrooxepin, perhydrooxepin, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dioxolane, dioxane, dioxaindane, benzodioxane, thiochromane, dihydrobenzodioxine, dihydrobenzoxathiine, chromane, pyrazolopyrimidine, imidazopyridazine, imidazopyridine, pyrrolopyridine, imidazopyrazine, pyrazolopyridine, pyrazolopyrimidine, imidazopyridine and triazolopyridine rings.

In the present invention, "a halogen" may include fluorine, chlorine, bromine and iodine.

In the present invention, "a C1-6 alkyl group" may include, for example, methyl, ethyl, n-propyl, isopropyl, n-butyl, sec-butyl, tert-butyl, isobutyl, pentyl, 1-methylbutyl, 2-methylbutyl, 3-methylbutyl, 1,1-dimethylpropyl, 1,2-dimethylpropyl, 2,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 1-methyl-1-ethylpropyl, 2-methyl-2-ethylpropyl, 1-ethylbutyl, and 2-ethylbutyl groups.

In the present invention, "a C2-6 alkenyl group" may include, for example, vinyl, 1-propenyl, 2-propenyl, 1-butenyl, 2-butenyl, 3-butenyl, 1-pentenyl, 2-pentenyl, 3-pentenyl, 4-pentenyl, 3-methyl-1-butenyl, 3-methyl-2-butenyl, 3-methyl-3-butenyl, 1-hexenyl, 2-hexenyl, 3-hexenyl, 4-hexenyl and 5-hexenyl groups.

In the present invention, "a C2-6 alkynyl group" may include, for example, ethynyl, 1-propynyl, 2-propynyl, 1-butynyl, 2-butynyl, 3-butynyl, 1-pentynyl, 2-pentynyl, 3-pentynyl, 4-pentynyl, 3-methyl-1-butynyl, 1-hexynyl, 2-hexynyl, 3-hexynyl, 4-hexynyl and 5-hexynyl groups.

In the present invention, "a C5-6 monocyclic carbocycle" may include, for example, cyclopentane, cyclohexane, cyclopentene, cyclohexene, cyclopentadiene, cyclohexadiene and benzene rings.

In the present invention, "a 5- to 6-membered monocyclic heterocycle" may include, for example, pyrrole, imidazole, triazole, tetrazole, pyrazole, pyridine, piperidine, piperazine, pyrazine, pyrimidine, pyridazine, furan, pyran, thiophene, thiopyran, oxazole, isoxazole, thiazole, isothiazole, furazan, oxadiazole, oxazine, oxadiazine, thiadiazole, thiazine, thiadiazine, pyrroline, pyrrolidine, imidazoline, imidazolidine, triazoline, triazolidine, tetrazoline, tetrazolidine, pyrazoline, pyrazolidine, dihydropyridine, tetrahydropyridine, dihydropyrazine, tetrahydropyrazine, dihydropyrimidine,

tetrahydropyrimidine, perhydropyrimidine, dihydropyridazine, tetrahydropyridazine, perhydropyridazine, dihydrofuran, tetrahydrofuran, dihydropyran, tetrahydropyran, dihydrothiophene, tetrahydrothiophene, dihydrothiopyran, tetrahydrothiopyran, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrothiadiazole, tetrahydrothiadiazole (thiadiazolidine), dihydrothiazine, tetrahydrothiazine, dihydrothiadiazine, tetrahydrothiadiazine, morpholine, thiomorpholine and oxathiane rings.

In the present invention, "a C1-3 alkyl group" includes methyl, ethyl, n-propyl and isopropyl groups.

In the present invention, "a C3-6 cycloalkyl group" includes cyclopropyl, cyclobutyl, cyclopentyl and cyclohexyl groups.

In the present invention, "a C1-6 alkoxy group" may include, for example, methoxy, ethoxy, propoxy, isopropoxy, butoxy, 1-methylpropoxy, tert-butoxy, isobutoxy, pentyloxy, 1-methylbutoxy, 2-methylbutoxy, 3-methylbutoxy, 1,1-dimethylpropoxy, 1,2-dimethylpropoxy, 2,2-dimethylpropoxy, hexyloxy, 1-methylpentyloxy, 2-methylpentyloxy, 3-methylpentyloxy, 4-methylpentyloxy, 1,1-dimethylbutoxy, 1,2-dimethylbutoxy, 1,3-dimethylbutoxy, 1-methyl-1-ethylpropoxy, 1-methyl-2-ethylpropoxy, 1,2-dimethylbutoxy, 2,2-dimethylbutoxy, 1-ethyl-2-methylpropoxy, 2-ethyl-2-methylpropoxy and 1-ethylbutoxy groups.

In the present invention, a "C1-3 alkoxy group" may include, for example, methoxy, ethoxy, n-propoxy, and isopropoxy groups.

In the present invention, "a 3- to 7-membered monocyclic heterocycle" may include, for example, aziridine, oxetane, azetidine, pyrrole, imidazole, triazole, tetrazole, pyrazole, pyridine, piperidine, piperazine, pyrazine, pyrimidine, pyridazine, azepine, diazepine, furan, pyran, oxepin, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, pyrroline, pyrrolidine, imidazoline, imidazolidine, triazoline, triazolidine, tetrazoline, tetrazolidine, pyrazoline, pyrazolidine, dihydropyridine, tetrahydropyridine, dihydropyrazine, tetrahydropyrazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrofuran, tetrahydrofuran, dihydropyran, tetrahydropyran, dihydrooxepin, tetrahydrooxepin, perhydrooxepin, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine and oxathiane rings.

In the present invention, "a 5- to 10-membered monocyclic aromatic heterocycle or bicyclic aromatic heterocycle" may include, for example, pyrrole, imidazole, triazole, tetrazole, pyrazole, pyridine, pyrazine, pyrimidine, pyridazine, azepine, diazepine, furan, oxepin, thiophene, thiepine, oxazole, isoxazole, thiazole, isothiazole, furazan, oxadiazole, oxazepine, oxadiazepine, thiadiazole, indole, isoindole, indolizine, benzofuran, isobenzofuran, benzothiophene, isobenzothiophene, indazole, quinoline, isoquinoline, quinolidine, purine, phthalazine, pteridine, naphthyridine, quinoxaline, quinazoline, cinnoline, benzoxazole, benzothiazole, benzimidazole, benzofurazan, benzothiadiazole, benzotriazole, pyrazolopyrimidine, imidazopyridazine, imidazopyridine, pyrrolopyridine, imidazopyrazine, pyrazolopyridine, pyrazolopyrimidine, imidazopyridine and triazolopyridine rings.

In the present invention, a "C5-6 monocyclic aromatic carbocycle" include, for example, benzene.

In the present invention, "a 5- to 6-membered monocyclic aromatic heterocycle" may include, for example, pyrrole, imidazole, triazole, tetrazole, pyrazole, pyridine, pyrazine, pyrimidine, pyridazine, furan, thiophene, oxazole, isoxazole, thiazole, isothiazole, furazan, oxadiazole and thiadiazole rings.

In the present invention, the phrase "when, further, two $R_5$ groups are each independently a C1-3 alkyl group or a hydroxy group and the $R_5$ groups are attached to carbon atoms adjacent to each other on the C5-6 monocyclic carbocycle or the 5- to 6-membered monocyclic heterocycle, the $R_5$ groups may together form a ring" may indicate, for example, the following groups:

[C7]

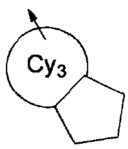 , 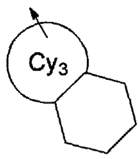 , 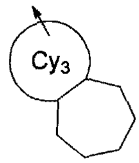 , 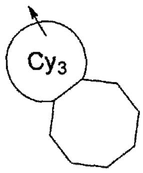 ,

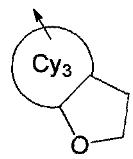 , and 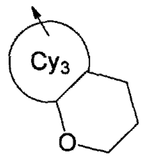

(wherein a ring $Cy_3$ represents a C5-6 monocyclic carbocycle or a 5- to 6-membered monocyclic heterocycle and an arrow means binding to the ring $Cy_1$).

In the present invention, the phrase "$R_5$ is $-SO_2NR_{18}R_{19}$ and when $R_{18}$ and $R_{19}$ are each independently a C1-6 alkyl group, $R_{18}$ and $R_{19}$ may together form a ring" may indicate, for example, the following groups:

[C8]

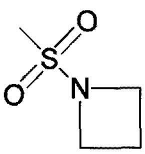 , 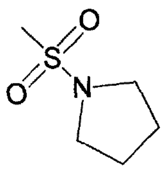 , 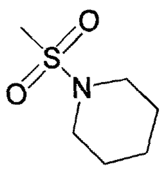 , and 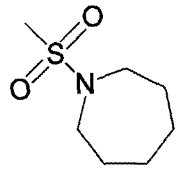

In the present invention, the ring $Cy_1$ is preferably a C5-6 monocyclic carbocycle or a 5- to 6-membered monocyclic heterocycle.

In the present invention, the ring $Cy_1$ is more preferably cyclopentane, cyclohexane, benzene, pyran, thiopyran, pyrrolidine, piperidine, piperazine, imidazoline, imidazolidine, morpholine, thiomorpholine or a 5- to 6-membered monocyclic aromatic heterocycle.

In the present invention, the ring $Cy_1$ is further preferably benzene or a 5- to 6-membered monocyclic aromatic heterocycle.

In the present invention, the ring $Cy_1$ is still more preferably a benzene, pyrrole, imidazole, pyrazole, pyridine, pyrazine, pyrimidine, pyridazine, furan, thiophene, oxazole, isoxazole, thiazole or isothiazole ring.

In the present invention, the ring $Cy_1$ is yet more preferably a benzene, imidazole, pyrazole, pyridine, pyrazine, pyrimidine or pyridazine ring.

In the present invention, the ring $Cy_1$ is yet still more preferably a benzene, pyrazole or pyridine ring.

In the present invention, the ring $Cy_1$ is the most preferably a benzene or pyridine ring.

In the present invention, the ring $Cy_2$ is preferably a 5- to 10-membered monocyclic aromatic heterocycle or bicyclic aromatic heterocycle.

In the present invention, the ring $Cy_2$ is more preferably a pyridine, pyrazine, pyrimidine, pyridazine, indole, isoindole, indolizine, benzofuran, isobenzofuran, benzothiophene, isobenzothiophene, indazole, quinoline, isoquinoline, quinolidine, purine, phthalazine, pteridine, naphthyridine, quinoxaline, quinazoline, cinnoline, benzoxazole, benzothiazole, benzimidazole, benzofurazan, benzothiadiazole, benzotriazole, pyrazolopyrimidine, imidazopyridazine, imidazopyridine, pyrrolopyridine, imidazopyrazine, pyrazolopyridine, pyrazolopyrimidine, imidazopyridine or triazolopyridine ring.

In the present invention, the ring $Cy_2$ is still more preferably a pyridine, pyrazine, pyrimidine, pyridazine, indole, isoindole, indolizine, indazole, quinoline, isoquinoline, quinolidine, purine, phthalazine, pteridine, naphthyridine, quinoxaline, quinazoline, cinnoline, benzoxazole, benzothiazole, benzimidazole, benzofurazan, benzothiadiazole, benzotriazole, pyrazolopyrimidine, imidazopyridazine, imidazopyridine, pyrrolopyridine, imidazopyrazine, pyrazolopyridine, pyrazolopyrimidine, imidazopyridine or triazolopyridine ring.

In the present invention, the ring $Cy_2$ is yet more preferably a pyridine, pyrazine, pyrimidine, pyridazine, pyrazolopyrimidine, imidazopyridazine, imidazopyridine, pyrrolopyridine, imidazopyrazine, pyrazolopyridine, pyrazolopyrimidine, imidazopyridine or triazolopyridine ring.

In the present invention, the ring $Cy_2$ is yet still more preferably a pyridine, pyrimidine, pyrazolopyrimidine, imidazopyridazine, imidazopyridine, pyrrolopyridine, imidazopyrazine or pyrazolopyridine ring.

In the present invention, the ring $Cy_2$ is the most preferably a pyridine or pyrazolopyrimidine ring.

In the present invention, $R_1$ is preferably (1) a halogen, (2) a C1-3 alkyl group optionally substituted with a halogen, (3) a benzene ring optionally substituted with one or two Rs groups, (4) a 5- to 6-membered monocyclic aromatic heterocycle optionally substituted with one or two $R_5$ groups, (5) a methylsulfonyl group or (6) N,N-dimethylsulfonamide.

In the present invention, $R_1$ is more preferably (1) a halogen, (2) a methyl group, (3) a trifluoromethyl group, (4) a difluoromethyl group, (5) a monofluoromethyl group, (6) a trichloromethyl group, (7) a dichloromethyl group, (8) a monochloromethyl group, (9) a benzene ring optionally substituted with one or two $R_5$ groups, (10) a pyrrole, imidazole, triazole, tetrazole, pyrazole, pyridine, pyrazine, pyrimidine, pyridazine, furan, thiophene, oxazole, isoxazole, thiazole, isothiazole, furazan, oxadiazole or thiadiazole ring optionally substituted with one or two $R_5$ groups, (11) a methylsulfonyl group or (12) N,N-dimethylsulfonamide.

In the present invention, $R_1$ is still more preferably (1) a halogen, (2) a methyl group, (3) a trifluoromethyl group, (4) a difluoromethyl group, (5) a monofluoromethyl group, (6) a benzene ring, (7) an indane ring, (8) a tolyl group, (9) a dimethylbenzene ring, (10) an imidazole, triazole, pyrazole or pyridine ring optionally substituted with one or two $R_5$ groups or (11) a methylsulfonyl group.

In the present invention, $R_1$ is yet more preferably (1) a halogen, (2) a trifluoromethyl group, (3) a difluoromethyl group, (4) a benzene ring, (5) an indane ring, (6) a tolyl group, (7) a dimethylbenzene ring, (8) an imidazole, triazole, pyrazole or pyridine ring optionally substituted with one or two methyl, difluoromethyl or trifluoromethyl groups or (9) a methylsulfonyl group.

In the present invention, $R_1$ is yet still more preferably (1) a trifluoromethyl group, (2) a difluoromethyl group, (3) a benzene ring, (4) a triazole, pyrazole or pyridine ring optionally substituted with one or two methyl, difluoromethyl or trifluoromethyl groups or (5) a methylsulfonyl group.

In the present invention, $R_1$ is the most preferably (1) a trifluoromethyl group or (2) a triazole, pyrazole or pyridine ring optionally substituted with one or two methyl, difluoromethyl or trifluoromethyl groups.

In the present invention, $R_5$ is preferably (1) a halogen, (2) a methyl group optionally substituted with a halogen or (3) a C1-3 alkyl group optionally substituted with a hydroxy group or an oxo group.

In the present invention, $R_5$ is more preferably a methyl group, a trifluoromethyl group, a difluoromethyl group, an acetyl group or a hydroxyethyl group.

In the present invention, $R_5$ is the most preferably a methyl group, a trifluoromethyl group or a difluoromethyl group.

In the present invention, $R_2$ is preferably (1) a halogen, (2) a C1-3 alkyl group optionally substituted with a halogen or a hydroxy group, (3) a C3-6 cycloalkyl group, (4) a C1-3 alkoxy group, (5) an amino group, (6) a methylamino, ethylamino, n-propylamino, isopropylamino, n-butylamino, sec-butylamino, tert-butylamino, isobutylamino or dimethylamino group optionally substituted with a hydroxy group, (7) a 3- to 7-membered monocyclic heterocycle or (8) -O-(3- to 7-membered monocyclic heterocycle).

In the present invention, $R_2$ is more preferably a halogen, a methyl group, a trifluoromethyl group, a difluoromethyl group, a monofluoromethyl group, a hydroxymethyl group, a hydroxyethyl group, a 2-methylhydroxyethyl group, a cyclopropyl group, a methoxy group, an ethoxy group, an amino group, a methylamino group, an ethylamino group, a dimethylamino group, a 2-methyl-2-hydroxypropylamino group, an oxetanyloxy group, an azetidine ring, a pyrrolidine ring or a piperidine ring.

In the present invention, $R_2$ is still more preferably a halogen, a methyl group, a cyclopropyl group, a methoxy group, an amino group, a dimethylamino group, an oxetanyloxy group, an azetidine ring, a pyrrolidine ring or a piperidine ring.

In the present invention, $R_2$ is yet more preferably a halogen, a methyl group, an amino group, an azetidine ring or a pyrrolidine ring.

In the present invention, $R_2$ is the most preferably fluorine, chlorine, a methyl group, an amino group or an azetidine ring.

In the present invention, $R_3$ is preferably hydrogen or fluorine and the most preferably hydrogen.

In the present invention, $R_4$ is preferably hydrogen or fluorine and the most preferably hydrogen.

In the present invention, $R_6$ is preferably a C1-3 alkyl group optionally substituted with a halogen.

In the present invention, $R_6$ is more preferably a methyl group, an ethyl group or a n-propyl group.

In the present invention, preferably $R_7$ and $R_8$ are each independently a hydrogen atom or a C1-3 alkyl group optionally substituted with a hydroxy group.

In the present invention, more preferably $R_7$ and $R_8$ are each independently a hydrogen atom, a methyl group, an ethyl group, a n-propyl group, an isopropyl group or a 2-hydroxypropyl group.

In the present invention, still more preferably $R_7$ and $R_8$ are each independently a hydrogen atom, a methyl group, an ethyl group or a n-propyl group.

In the present invention, $R_9$ is preferably a hydrogen atom, a methyl group or an ethyl group.

In the present invention, preferably $R_{10}$ to $R_{16}$ are each independently a hydrogen atom, a methyl group, an ethyl group or a n-propyl group.

In the present invention, $R_{17}$ is preferably a C1-3 alkyl group optionally substituted with a halogen.

In the present invention, $R_{17}$ is more preferably a methyl group, an ethyl group or a n-propyl group.

In the present invention, preferably $R_{18}$ and $R_{19}$ are each independently a hydrogen atom or a C1-3 alkyl group optionally substituted with a hydroxy group.

In the present invention, more preferably $R_{18}$ and $R_{19}$ are each independently a hydrogen atom, a methyl group, an ethyl group, a n-propyl group, an isopropyl group or a 2-hydroxypropyl group.

In the present invention, still more preferably $R_{18}$ and $R_{19}$ are each independently a hydrogen atom, a methyl group, an ethyl group or a n-propyl group.

In the present invention, $R_{20}$ is preferably a hydrogen atom, a methyl group or an ethyl group.

In the present invention, preferably $R_{21}$ to $R_{29}$ are each independently a hydrogen atom, a methyl group, an ethyl group or a n-propyl group.

In the present invention, m1 is preferably an integer of 2.

In the present invention, m2 is preferably an integer of 2.

In the present invention, p is preferably an integer of 0 to 3.

In the present invention, q is preferably an integer of 0 to 3.

In the present invention, r is preferably an integer of 0 to 1.

In the present invention, the General Formula (I) is preferably those having the combinations of preferable definitions for the ring $Cy_1$, the ring $Cy_2$, $R_1$, $R_2$, $R_3$, $R_4$, $R_5$, m1, m2, p, q and r.

In the present invention, the compound represented by General Formula (I), a salt thereof, an N-oxide thereof, a solvate thereof, or a prodrug thereof is preferably a compound represented by General Formula (I-A):

[C9]

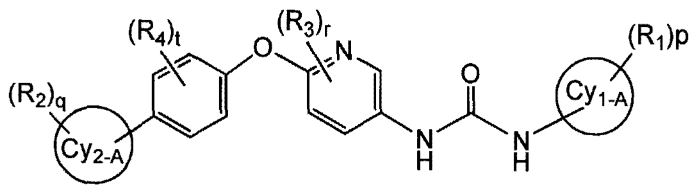

(I-A)

wherein, the ring $Cy_{1-A}$ represents a C5-6 monocyclic aromatic carbocycle, the ring $Cy_{2-A}$ represents a 5- to 10-membered monocyclic aromatic heterocycle or bicyclic aromatic heterocycle, t represents an integer of 0 to 4 (preferably an integer of 0 to 2, and the other symbols have the same meanings as above, provided that, when t represents an integer of 2 or more, $R_4$ groups may each independently be the same or different from each other, a salt thereof, an N-oxide thereof, a solvate thereof, or a prodrug thereof.

In addition, in another aspect, the compound represented by General Formula (I), a salt thereof, an N-oxide thereof, a solvate thereof, or a prodrug thereof is preferably a compound represented by General Formula (I-B):

[C10]

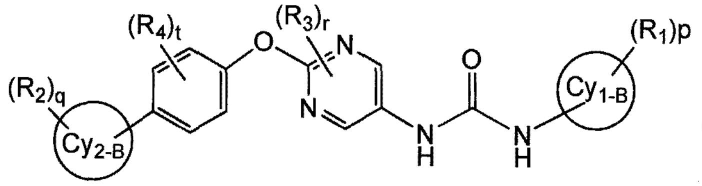

(I-B)

wherein, the ring $Cy_{1-B}$ represents a C5-6 monocyclic aromatic carbocycle, the ring $Cy_{2-B}$ represents a 5- to 10-membered monocyclic aromatic heterocycle or bicyclic aromatic heterocycle, and the other symbols have the same meanings as above, a salt thereof, an N-oxide thereof, a solvate thereof, or a prodrug thereof.

In addition, in another aspect, the compound represented by General Formula (I), a salt thereof, an N-oxide thereof, a solvate thereof, or a prodrug thereof is preferably a compound represented by General Formula (I-a) or General Formula (I-b):

[C11]

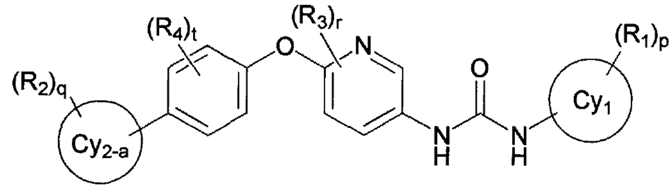

(I-a)

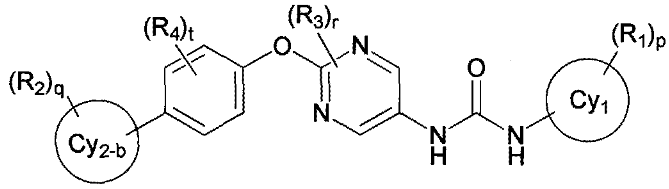

(I-b)

wherein, the ring $Cy_{2-a}$ and the ring $Cy_{2-b}$ represent a 5-to 10-membered monocyclic aromatic heterocycle or bicyclic aromatic heterocycle, and the other symbols have the same meanings as above, a salt thereof, an N-oxide thereof, a solvate thereof, or a prodrug thereof.

In the present invention, the compound represented by General Formula (I), a salt thereof, an N-oxide thereof, a solvate thereof, or a prodrug thereof is more preferably a compound represented by General Formula (I-c) or General Formula (I-d):

[C12]

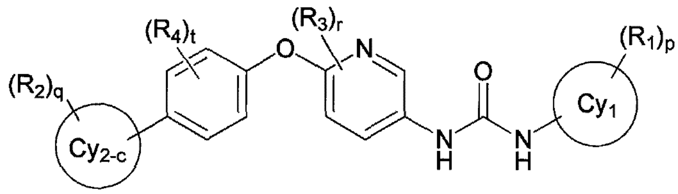

(I-c)

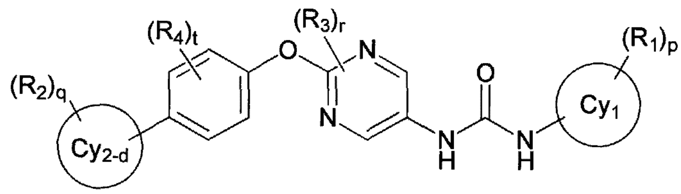

(I-d)

wherein, the ring $Cy_{2-c}$ and the ring $Cy_{2-d}$ represent a pyridine ring, a pyrimidine ring, a pyrazolopyrimidine ring, an imidazopyridazine ring, an imidazopyridine ring, a pyrrolopyridine ring, an imidazopyrazine ring, or a pyrazolopyridine ring, and the other symbols have the same meanings as above, a salt thereof, an N-oxide thereof, a solvate thereof, or a prodrug thereof.

In addition, in another aspect, the compound represented by General Formula (I), a salt thereof, an N-oxide thereof, a solvate thereof, or a prodrug thereof is preferably a compound represented by General Formula (I-e) or General Formula (I-f):

[C13]

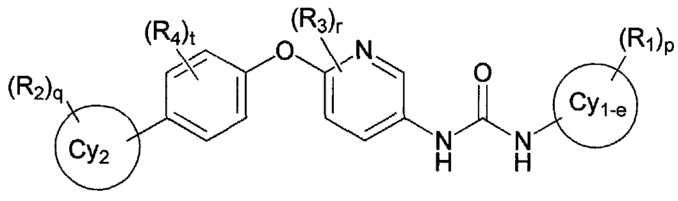

(I-e)

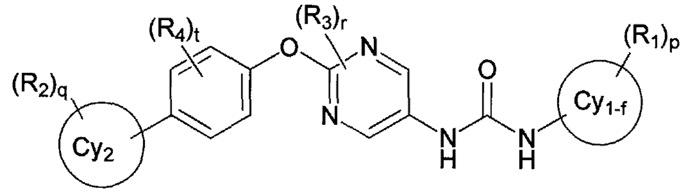

(I-f)

wherein, the ring $Cy_{1-e}$ and the ring $Cy_{1-f}$ represent a benzene ring or a 5- to 6-membered monocyclic aromatic heterocycle, and the other symbols have the same meanings as above, a salt thereof, an N-oxide thereof, a solvate thereof, or a prodrug thereof.

In the present invention, the compound represented by General Formula (I), a salt thereof, an N-oxide thereof, a solvate thereof, or a prodrug thereof is more preferably a compound represented by General Formula (I-g) or General Formula (I-h):

[C14]

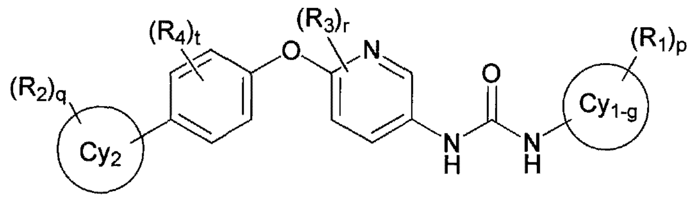

(I-g)

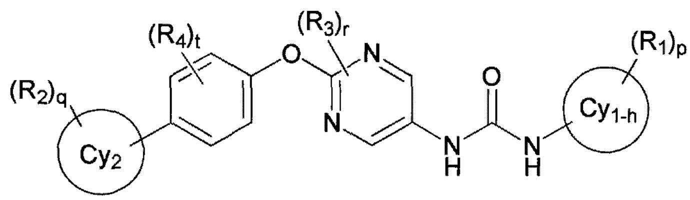

(I-h)

wherein, the ring $Cy_{1-g}$ and the ring $Cy_{1-h}$ represent a benzene ring, a pyridine ring, or a pyrazole ring, and the other symbols have the same meanings as above, a salt thereof, an N-oxide thereof, a solvate thereof, or a prodrug thereof.

In addition, in another aspect, the compound represented by General Formula (I), a salt thereof, an N-oxide thereof, a solvate thereof, or a prodrug thereof is preferably a compound represented by General Formula (I-j) or General Formula (I-k):

[C15]

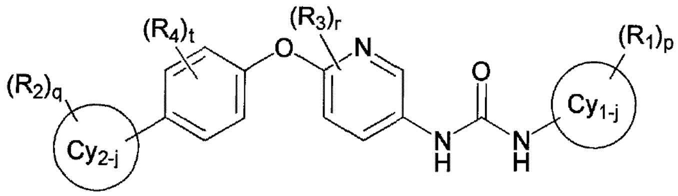

(I-j)

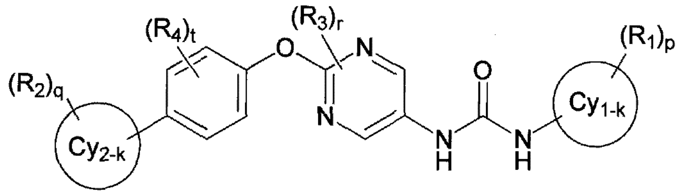

(I-k)

wherein, the ring $Cy_{2-j}$ and the ring $Cy_{2-k}$ represent a 5-to 10-membered monocyclic aromatic heterocycle or bicyclic aromatic heterocycle, the ring $Cy_{1-j}$ and the ring $Cy_{1-k}$ represent a benzene ring or a 5- to 6-membered monocyclic aromatic heterocycle, and the other symbols have the same meanings as above, a salt thereof, an N-oxide thereof, a solvate thereof, or a prodrug thereof.

In the present invention, the compound represented by General Formula (I), a salt thereof, an N-oxide thereof, a solvate thereof, or a prodrug thereof is more preferably a compound represented by General Formula (I-m) or General Formula (I-n):

[C16]

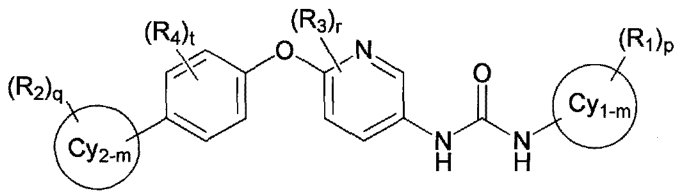

(I-m)

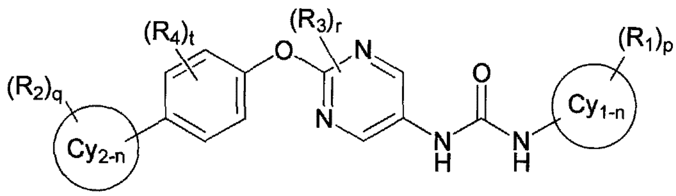

(I-n)

wherein, the ring $Cy_{2-m}$ and the ring $Cy_{2-n}$ represent a pyridine ring, a pyrimidine ring, a pyrazolopyrimidine ring, an imidazopyridazine ring, an imidazopyridine ring, a pyrrolopyridine ring, an imidazopyrazine ring, or a pyrazolopyridine ring, the ring $Cy_{1-m}$ and the ring $Cy_{1-n}$ represent a benzene ring, a pyridine ring, or a pyrazole ring, and the other symbols have the same meanings as above, a salt thereof, an N-oxide thereof, a solvate thereof, or a prodrug thereof.

In addition, in another aspect, in the present invention, the compound represented by General Formula (I), a salt thereof, an N-oxide thereof, a solvate thereof, or a prodrug thereof is preferably a compound represented by General Formula (I-i) or General Formula (I-ii):

[C17]

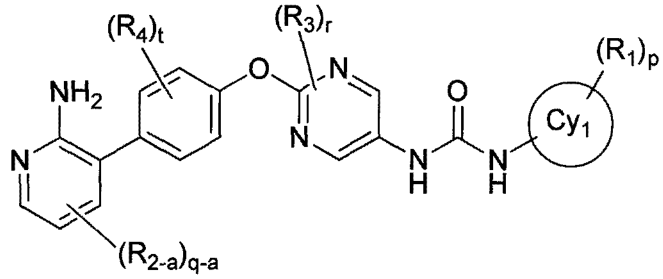

(I-i)

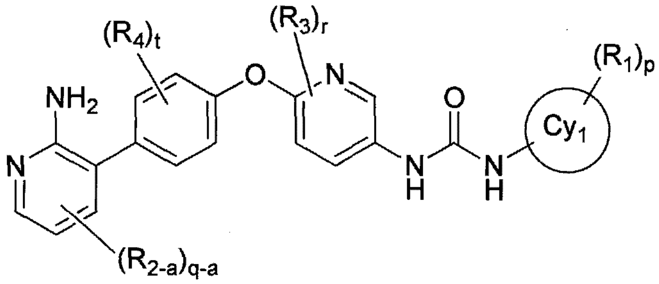

(I-ii)

wherein, $R_{2\text{-}a}$ represents the same meaning as $R_2$, and q-a represents an integer of 0 to 3 (preferably, an integer of 0 to 1), and the other symbols have the same meanings as above, provided that, when q-a represents an integer of 2 or more, $R_{2\text{-}b}$ groups may each independently be the same or different from each other, a salt thereof, an N-oxide thereof, a solvate thereof, or a prodrug thereof.

In the present invention, a compound represented by General Formula (I-i) or General Formula (I-ii), a salt thereof, an N-oxide thereof, a solvate thereof, or a prodrug thereof is more preferably a compound represented by General Formula (I-i-a) or General Formula (I-ii-b):

[C18]

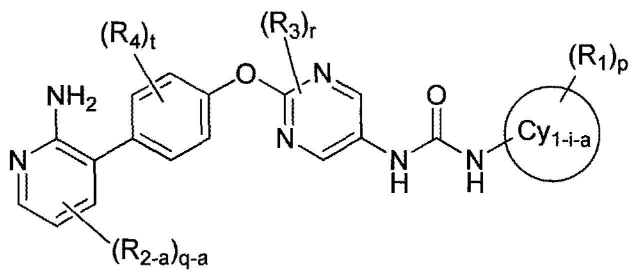

(I-i-a)

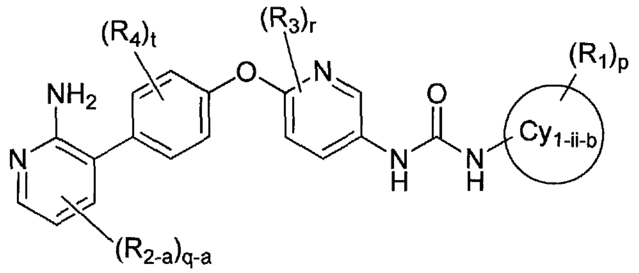

(I-ii-b)

wherein, the ring $Cy_{1\text{-}i\text{-}a}$ and the ring $Cy_{1\text{-}ii\text{-}b}$ represent a benzene ring or a 5- to 6-membered monocyclic aromatic heterocycle, and the other symbols have the same meanings as above, a salt thereof, an N-oxide thereof, a solvate

thereof, or a prodrug thereof.

In the present invention, a compound represented by General Formula (I-i) or General Formula (I-ii), a salt thereof, an N-oxide thereof, a solvate thereof, or a prodrug thereof is still more preferably a compound represented by General Formula (I-i-c) or General Formula (I-ii-d):

[C19]

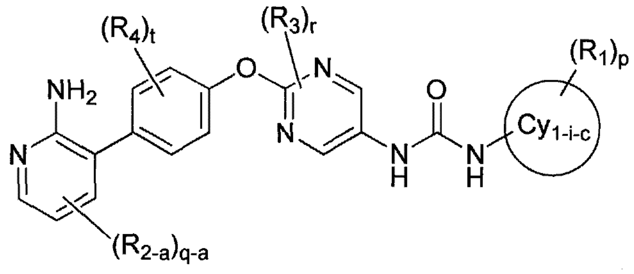

(I-i-c)

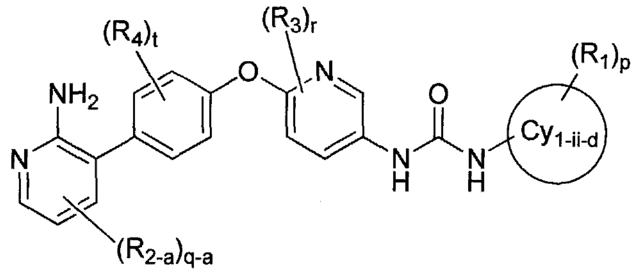

(I-ii-d)

wherein, the ring $Cy_{1-i-c}$ and the ring $Cy_{1-ii-d}$ represent a benzene ring, a pyridine ring, or a pyrazole ring, and the other symbols have the same meanings as above, a salt thereof, an N-oxide thereof, a solvate thereof, or a prodrug thereof.

In addition, in another aspect, in the present invention, the compound represented by General Formula (I), a salt thereof, an N-oxide thereof, a solvate thereof, or a prodrug thereof is preferably a compound represented by General Formula (I-iii) or General Formula (I-iv):

[C20]

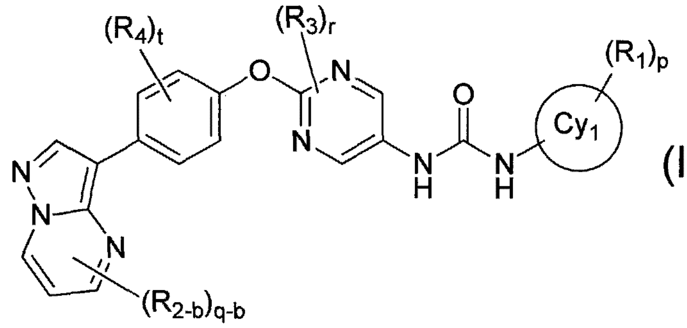

(I-iii)

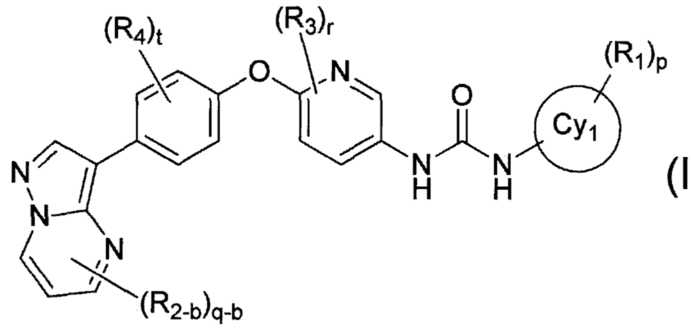

(I-iv)

wherein, $R_{2-b}$ represents the same meaning as $R_2$, and q-b represents an integer of 0 to 4 (preferably, an integer of 0 to 1), and the other symbols have the same meanings as above, provided that, when q-b represents an integer of 2 or more, $R_{2-b}$ groups may each independently be the same or different from each other, a salt thereof, an N-oxide thereof, a solvate thereof, or a prodrug thereof.

In the present invention, a compound represented by General Formula (I-iii) or General Formula (I-iv), a salt thereof, an N-oxide thereof, a solvate thereof, or a prodrug thereof is more preferably a compound represented by General Formula (I-iii-a) or General Formula (I-iv-b):

[C21]

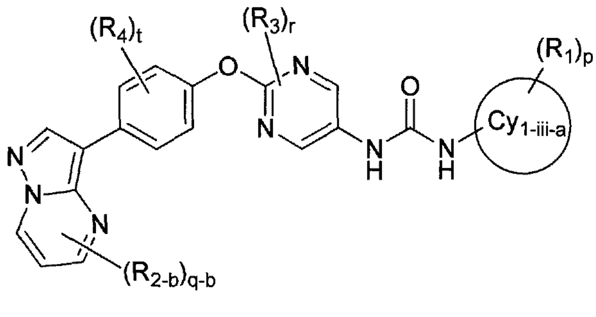

(I-iii-a)

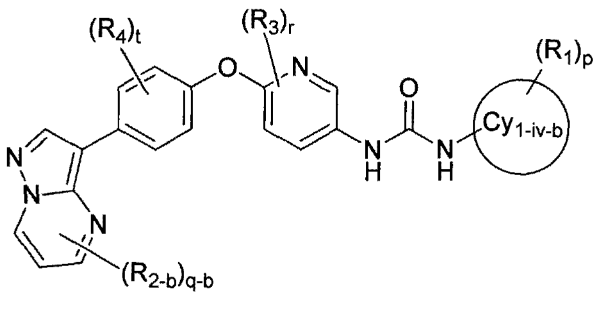

(I-iv-b)

wherein, the ring $Cy_{1-iii-a}$ and the ring $Cy_{1-iv-b}$ represent a benzene ring or a 5- to 6-membered monocyclic aromatic

heterocycle, and the other symbols have the same meanings as above, a salt thereof, an N-oxide thereof, a solvate thereof, or a prodrug thereof.

In the present invention, a compound represented by General Formula (I-iii) or General Formula (I-iv), a salt thereof, an N-oxide thereof, a solvate thereof, or a prodrug thereof is still more preferably a compound represented by General Formula (I-iii-c) or General Formula (I-iv-d):

[C22]

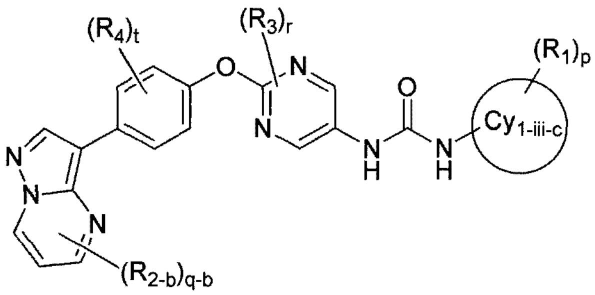

(I-iii-c)

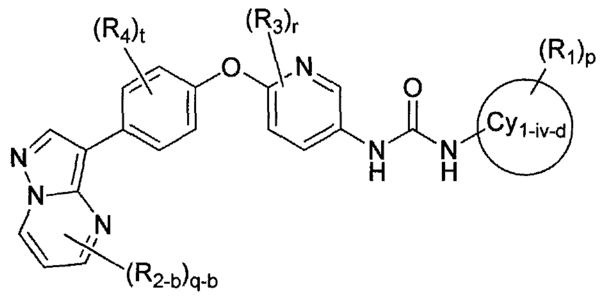

(I-iv-d)

wherein, the ring $Cy_{1\text{-iii-c}}$ and the ring $Cy_{1\text{-iv-d}}$ represent a benzene ring or a pyridine ring, and the other symbols have the same meanings as above, a salt thereof, an N-oxide thereof, a solvate thereof, or a prodrug thereof.

In the present invention, the compound represented by General Formula (I), a salt thereof, an N-oxide thereof, a solvate thereof, or a prodrug thereof is preferably

(1) 1-(2-(1H-pyrazol-1-yl)-5-(trifluoromethyl)phenyl)-3-(2-(4-(2-amino-5-chloropyridin-3-yl)phenoxy)pyrimidin-5-yl)urea,

(2) 1-(2-(4-(2-amino-5-chloropyridin-3-yl)phenoxy)pyrimidin-5-yl)-3-(2-(4-methyl-1H-1,2,3-triazol-1-yl)-5-(trifluoromethyl)phenyl)urea,

(3) 1-(2-(4-(2-amino-5-chloropyridin-3-yl)phenoxy)pyrimidin-5-yl)-3-(5-(trifluoromethyl)-2-(3-(trifluoromethyl)-1H-pyrazol-1-yl) phenyl)urea,

(4) 1-(2-(4-(2-amino-5-chloropyridin-3-yl)phenoxy)pyrimidin-5-yl)-3-(2-chloro-5-(trifluoromethyl)phenyl)urea,

(5) 1-(2-(1H-pyrazol-1-yl)-5-(trifluoromethyl)phenyl-3-(6-(4-(2-amino-5-chloropyridin-3-yl)phenoxy)pyridin-3-yl)urea,

(6) 1-(2-(1H-1,2,3-triazol-1-yl)-5-(trifluoromethyl)phenyl-3-(6-(4-(2-amino-5-chloropyridin-3-yl)phenoxy)pyridin-3-yl)urea,

(7) 1-(6-(4-(2-amino-5-chloropyridin-3-yl)phenoxy)pyridin-3-yl)-3-(2-(pyridin-3-yl)-5-(trifluoromethyl)phenyl)urea,

(8) 1-(2-(4-(2-amino-5-chloropyridin-3-yl)phenoxy)pyrimidin-5-yl)-3-(2-(1-methyl-1H-pyrazol-5-yl)-5-(trifluoromethyl)phenyl)urea,

(9) 1-(2-(1H-1,2,3-triazol-1-yl)-5-(trifluoromethyl)phenyl)-3-(2-(4-(2-amino-5-fluoropyridin-3-yl)phenoxy)pyrimidin-5-yl)urea,

(10) 1-(2-(4-(2-amino-5-fluoropyridin-3-yl)phenoxy)pyrimidin-5-yl)-3-(2-(pyridin-3-yl)-5-(trifluoromethyl)phenyl)urea,

(11) 1-(2-(1H-pyrazol-1-yl)-4-(trifluoromethyl)phenyl)-3-(2-(4-(2-amino-5-chloropyridin-3-yl)phenoxy)pyrimidin-5-yl)urea,

(12) 1-(2-(4-(2-amino-5-chloropyridin-3-yl)phenoxy)pyrimidin-5-yl)-3-(2-fluoro-4-(trifluoromethyl)phenyl)urea,

(13) 1-(2-(4-(2-amino-5-chloropyridin-3-yl)phenoxy)pyrimidin-5-yl)-3-(2-chloro-4-(trifluoromethyl)phenyl)urea,

(14) 1-(2-14-[2-amino-5-(trifluoromethyl)-3-pyridinyl]phenoxy}-5-pyrimidinyl)-3-{5-(trifluoromethyl)-2-[3-(trifluoromethyl)-1H-pyrazol-1-yl]phenyl}urea,

(15) 1-{2-[4-(2-amino-5-chloro-3-pyridinyl)phenoxy]-5-pyrimidinyl}-3-[4-(trifluoromethyl)-2-biphenylyl]urea,

(16) 1-{2-[4-(2-amino-5-fluoro-3-pyridinyl)phenoxy]-5-pyrimidinyl}-3-[4-(trifluoromethyl)-2-biphenylyl]urea,

(17) 1-{2-[4-(2-amino-5-chloro-3-pyridinyl)phenoxy]-5-pyrimidinyl}-3-[2-(4-chloro-1H-pyrazol-1-yl)-5-(trifluoromethyl)phenyl]urea,

(18) 1-{2-[4-(2-amino-5-chloro-3-pyridinyl)phenoxy]-5-pyrimidinyl}-3-{5-chloro-2-[3-(trifluoromethyl)-1H-pyrazol-1-yl]phenyl}urea,

(19) 1-{2-[4-(2-amino-5-chloro-3-pyridinyl)phenoxy]-5-pyrimidinyl}-3-[2,4-bis(trifluoromethyl)phenyl]urea,

(20) 1-(2-{4-[2-amino-5-(trifluoromethyl)-3-pyridinyl]phenoxy}-5-pyrimidinyl)-3-[2-(4-chloro-1H-pyrazol-1-yl)-5-(trifluoromethyl)phenyl]urea,

(21) 1-{2-[4-(2-amino-5-fluoro-3-pyridinyl)phenoxy]-5-pyrimidinyl}-3-[2-(methylsulfonyl)-5-(trifluoromethyl)phenyl]urea,

(22) 1-(2-(4-[2-amino-S-(trifluoromethyl)-3-pyridinyl]phenoxy}-5-pyrimidinyl)-3-[2-(methylsulfonyl)-5-(trifluoromethyl)phenyl]urea,

(23) 1-{2-[4-(2-amino-5-chloro-3-pyridinyl)phenoxy]-5-pyrimidinyl}-3-[2-(methylsulfonyl)-5-(trifluoromethyl)phenyl]urea,

(24) 2-{[(2-{4-[2-amino-5-(trifluoromethyl)-3-pyridinyl]phenoxy}-5-pyrimidinyl)carbamoyl]amino}-N,N-dimethyl-4-(trifluoromethyl)benzenesulfonamide,

(25) 1-(2-(4-(5-(azetidin-1-yl)pyrazolo[1,5-alpyrimidin-3-yl)phenoxy)pyrimidin-5-yl)-3-(2-(pyridin-3-yl)-5-(trifluoromethyl)phenyl)urea,

(26) 1-(2-(4-(5-(azetidin-1-yl)pyrazolo[1,5-a]pyrimidin-3-yl)phenoxy)pyrimidin-5-yl)-3-(2-(1-methyl-1H-pyrazol-5-yl)-5-(trifluoromethyl)phenyl)urea,

(27) 1-(2-(4-(5-methoxypyrazolo[1,5-a]pyrimidin-3-yl)phenoxy)pyrimidin-5-yl)-3-(2-(pyridin-3-yl)-5-(trifluoromethyl)phenyl)urea,

(28) 1-(2-(4-(pyrazolo[1,5-a]pyrimidin-3-yl)phenoxy)pyrimidin-5-yl)-3-(2-(pyridin-3-yl)-5-(trifluoromethyl)phenyl)urea,

(29) 1-(2-{4-[5-(dimethylamino)pyrazolo[1,5-a]pyrimidin-3-yl]phenoxy}-5-pyrimidiny1)-3-[2-(3-pyridinyl)-5-(trifluoromethyl)phenyl]urea,

(30) 1-(2-{4-[5-(dimethylamino)pyrazolo[1,5-a]pyrimidin-3-yl]phenoxy}-5-pyrimidinyl)-3-[2-(1-methyl-1H-pyrazol-5-yl)-5-(trifluoromethyl)phenyl]urea,

(31) 1-{2-[4-(5-methylpyrazolo[1,5-a]pyrimidin-3-yl)phenoxy]-5-pyrimidinyl}-3-[2-(3-pyridinyl)-5-(trifluoromethyl)phenyl]urea,

(32) 1-(2-{4-[5-(ethylamino)pyrazolo[1,5-a]pyrimidin-3-yllphenoxy)-5-pyrimidinyl)-3-[3'-methyl-4-(trifluoromethyl)-2-biphenylyl] urea,

(33) 1-(2-{4-[5-(dimethylamino)pyrazolo[1,5-a]pyrimidin-3-yl]phenoxy}-5-pyrimidinyl)-3-[4-(trifluoromethyl)-2-biphenylyl] urea,

(34) 1-(2-{4-[5-(dimethylamino)pyrazolo[1,5-a]pyrimidin-3-yl]phenoxy}-5-pyrimidinyl)-3-[3'-methyl-4-(trifluoromethyl)-2-biphenylyl]urea, or

(35) 1-(2-(4-[5-(dimethylamino)pyrazolo[1,5-a]pyrimidin-3-yl]phenoxy}-5-pyrimidinyl)-3-[2'-methyl-4-(trifluoromethyl)-2-biphenylyl]urea, a salt thereof, an N-oxide thereof, a solvate thereof, or a prodrug thereof.

[Isomers]

All isomers are encompassed by the present invention unless specifically stated. For example, an alkyl group, an alkenyl group, an alkynyl group and an alkoxy group comprise linear and branched groups. Further, rings, isomers in fused rings (E, Z, cis and trans forms), isomers due to asymmetric carbons (R and S forms, $\alpha$ and $\beta$ forms, enantiomers, diastereomers), optically active substances with optical rotatory (D, L, d and 1 forms), polar substances by chromatographic separation (high-polarity substances, low-polarity substances), equilibrated compounds, rotational isomers, mixtures thereof with any proportions and racemic mixtures are all encompassed by the present invention. Isomers due to tautomeric properties are also encompassed by the present invention.

[Salts]

The compound represented by the General Formula (I) may be converted to a salt according to well known methods.

The salt is preferably a pharmaceutically acceptable salt.

The salt is preferably water soluble.

The salt may include, for example, acid addition salts, alkali metal salts, alkaline-earth metal salts, ammonium

salts and amine salts.

The acid addition salt may include, for example, inorganic acid salts such as hydrochlorides, hydrobromides, hydroiodides, sulfates, phosphates and nitrates and organic acid salts such as acetates, lactates, tartrates, benzoates, citrates, methanesulfonates, ethanesulfonates, trifluoroacetates, benzenesulfonates, toluenesulfonates, isethionates, glucuronates and gluconates.

The alkali metal salt may include, for example, potassium salt and sodium salt.

The alkaline-earth metal salt may include, for example, calcium salt and magnesium salt.

Examples of ammonium salts include tetramethylammonium salts.

Examples of amine salts include triethylamine salts, methylamine salts, dimethylamine salts, cyclopentylamine salts, benzylamine salts, phenethylamine salts, piperidine salts, monoethanolamine salts, diethanolamine salts, tris(hydroxymethyl)aminomethane salts, lysine salts, arginine salts, and N-methyl-D-glucamine salts.

In addition, the compound represented by General Formula (I) or a salt thereof can be converted into an N-oxide according to any method. The N-oxide represents the compound represented by General Formula (I) or a salt thereof in which a nitrogen atom is oxidized, and specifically represents the compound represented by General Formula (I) or a salt thereof in which $A_1$, $A_2$, $A_3$, $A_4$, $A_5$, or $A_6$ is =N- and the nitrogen atom is oxidized. Alternatively, the N-oxide represents the compound represented by General Formula (I) or a salt thereof in which $Cy_1$ or $Cy_2$ is a nitrogen-containing heterocycle and the nitrogen atom is oxidized. Further, the N-oxide represents the compound represented by General Formula (I) or a salt thereof in which an amino group is oxidized.

The compound represented by the General Formula (I) and a salt thereof may be converted to a solvate. The solvate is preferably non-toxic and water soluble. Appropriate solvates may include, for example, solvates with water or an alcoholic solvent (e.g., ethanol).

[Prodrugs]

A prodrug of the compound represented by the General Formula (I) refers to a compound that is converted to the compound represented by the General Formula (I) by in vivo reaction with an enzyme or gastric acid. The prodrug of the compound represented by the General Formula (I) may include, for example, compounds wherein an amino group in the compound represented by the General Formula (I) is acylated, alkylated or phosphated (e.g., compounds wherein an amino group in the compound represented by the General Formula (I) is derivatized to eicosanoyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl, acetoxymethyl or tert-butyl); compounds wherein a hydroxy group in the compound represented by the General Formula (I) is acylated, alkylated, phosphated or borated (e.g., compounds wherein a hydroxy group in the compound represented by the General Formula (I) is derivatized to acetyl, palmitoyl, propanoyl, pivaloyl, succinyl, fumaryl, alanyl or dimethylaminomethylcarbonyl); compounds wherein a carboxy group in the compound represented by the General Formula (I) is esterified or amidated (e.g., compounds wherein a carboxy group in the compound represented by the General Formula (I) is derivatized to ethyl ester, phenyl ester, carboxymethyl ester, dimethylaminomethyl ester, pivaloyloxymethyl ester, 1-{(ethoxycarbonyl)oxy}ethyl ester, phthalidyl ester, (5-methyl-2-oxo-1,3-dioxolen-4-yl)methyl ester, 1-{[(cyclohexyloxy)carbonyl]oxy}ethyl ester or methylamide) and the like. These compounds may be prepared according to the methods well known per se. The prodrug of the compound represented by the General Formula (I) may be a hydrate or non-hydrate. The prodrug of the compound represented by the General Formula (I) may be the one which is converted to the compound represented by the General Formula (I) under physiological conditions described in "Iyakuhin no Kaihatsu (Development of Medicines)", vol. 7, "Bunshi Sekkei (Molecular Designs)", Hirokawa Shoten Co., 1990, pp. 163-198.

The atoms constituting the compound represented by the General Formula (I) may respectively be substituted with isotopes thereof (e.g. , $^{2}H$, $^{3}H$, $^{13}C$, $^{14}C$, $^{15}N$, $^{16}N$, $^{17}O$, $^{18}O$, $^{35}S$, $^{36}Cl$, $^{77}Br$, $^{125}I$ and the like) .

The compound represented by General Formula (I), a salt thereof, an N-oxide thereof, a solvate thereof, or a prodrug thereof can be produced according to the method described in WO 2014/129431, the method described in WO 2016/027754, a known method or a method equivalent thereto. Here, a raw material compound may be used as a salt. Regarding such salts, those described as a pharmaceutically acceptable salt of General Formula (I) are used.

[Combination drug]

In this specification, an MEK inhibitor is a substance having inhibiting activity toward Mitogen Activated Protein Kinase Kinase. Examples of MEK inhibitors include trametinib, selumetinib, binimetinib, cobimetinib, and PD-0325901.

In this specification, a CDK4/6 inhibitor is a substance having inhibiting activity toward cyclin-dependent kinase (CDK)4 and/or 6. Examples of CDK4/6 inhibitors include palbociclib, ribociclib, and abemaciclib.

In this specification, an EGFR inhibitor is a substance having inhibiting activity toward epidermal growth factor receptor (EGFR) tyrosine kinase. Examples of EGFR inhibitors include gefitinib, erlotinib, neratinib, canertinib, afatinib, lapatinib, osimertinib, brigatinib, cetuximab and panitumumab.

In this specification, a JAK1/2 inhibitor is a substance having inhibiting activity toward janus kinase (JAK)1 and/or 2. Examples of JAK1/2 inhibitors include ruxolitinib and baricitinib.

[Cancer]

In this specification, cancer includes all solid cancers and non-solid cancers, and as certain aspect, blood cancer (for example, leukemia (for example, acute myeloid leukemia, chronic myeloid leukemia, acute lymphocytic leukemia, chronic lymphocytic leukemia)), malignant lymphoma(Hodgkin lymphoma, non-Hodgkin lymphoma (for example, T/NT cell-derived lymphoma (for example, peripheral T-cell lymphoma, adult T-cell leukemia), B cell-derived lymphoma (for example, follicular lymphoma, diffuse large B-cell lymphoma)), extranodal lymphoma (for example, primary central nervous system lymphoma, primary testicular lymphoma, primary gastric lymphoma), multiple myeloma, myelodysplastic syndrome, myeloproliferative syndrome, thymoma, head and neck cancer ((for example, oral squamous cell carcinoma, head and neck squamous cell cancer, nasopharyngeal cancer, laryngeal cancer, tongue cancer, acoustic neuroma), esophageal cancer, gastroesophageal junction cancer, esophageal adenocarcinoma, stomach cancer, colorectal cancer, colon cancer, rectal cancer, small intestine cancer, anal cancer (for example, anal canal cancer), liver cancer (for example, hepatocellular carcinoma), gallbladder cancer, bile duct cancer (for example, intrahepatic cholangiocarcinoma), biliary tract cancer, pancreatic cancer, thyroid cancer, parathyroid cancer, lung cancer (for example, non-small cell lung cancer (for example, squamous non-small cell lung cancer, non-squamous non-small cell lung cancer), small cell lung cancer), breast cancer, ovarian cancer (for example, serous ovarian cancer, ovarian clear cell adenocarcinoma), fallopian tube cancer, uterine cancer (for example, cervical cancer, endometrial cancer, endometrial cancer), vaginal cancer, vulvar cancer, penile cancer, renal cancer (for example, renal cell carcinoma, clear cell renal cell carcinoma), adrenal carcinoma, urothelial carcinoma (for example, bladder cancer, upper urinary tract cancer, ureteral cancer, renal pelvic cancer and urethral cancer), prostate cancer, testicular tumor (for example, germ cell tumor), bone/sarcoma (for example, Kaposi sarcomas, Ewing sarcoma, childhood rhabdomyosarcoma, uterine leiomyosarcoma, soft tissue sarcoma, chondrosarcoma, lung sarcoma, osteosarcoma, congenital fibrosarcoma), skin cancer (for example, uveal malignant melanoma, malignant melanoma (for example, malignant melanoma in the skin, oral mucosal epithelium or orbit), Merkel cell carcinoma), brain tumor (for example, meningioma, glioma (for example, glioblastoma, gliosarcoma)), spine tumor, squamous cell carcinoma, pleural mesothelioma, primary peritoneal carcinoma, salivary gland tumor, MASC (mammary analogue secretory carcinoma), neuroblastoma, medulloblastoma, ocular retinoblastoma, inflammatory myofibroblastic tumor, congenital mesodermal nephroma, neuroendocrine tumors, pediatric cancer or unknown primary cancer may be exemplified.

Examples of another aspect of cancer include NTRK gene positive cancer. The NTRK gene positive cancer refers to cancer in which expression of NTRK genes (including NTRK1 genes, NTRK2 genes and NTRK3 genes) or Trk proteins (including TrkA proteins, TrkB proteins, and TrkC proteins) can be confirmed. Examples of NTRK genes include wild type and mutant type NTRK1 genes, NTRK2 genes and NTRK3 genes. NTRK gene positive cancers include cancer in which Trk proteins are constantly activated due to overexpression of wild type NTRK genes or expression of mutant type NTRK genes.

Examples of a certain aspect of mutant type NTRK genes include NTRK fusion genes (including NTRK1 fusion genes, NTRK2 fusion genes, and NTRK3 fusion genes). Examples of NTRK1 fusion genes include MPRIP-NTRK1, CD74-NTRK1, RABGAP1L-NTRK1, TPM3-NTRK1, TPR-NTRK1, TFG-NTRK1, PPL-NTRK1, CHTOP-NTRK1, ARHGEF2-NTRK1, NFASC-NTRK1, BCAN-NTRK1, LMNA-NTRK1 and TP53-NTRK1. Examples of NTRK2 fusion genes include QKI-NTRK2, NACC2-NTRK2, VCL-NTRK2, AGBL4-NTRK2, TRIM24-NTRK2, PAN3-NTRK2, AFAP1-NTRK2 and SQSTM1-NTRK2. Examples of NTRK3 fusion genes include ETV6-NTRK3, BTB1-NTRK3, LYN-NTRK3 and RBPMS-NTRK3.

NTRK fusion genes include NTRK fusion genes having a point mutation including G595R mutation, G667C mutation, F589L mutation, G639R mutation, G623R mutation, G696A mutation, and the like.

The G595R mutation refers to a mutation in which, in wild type or mutant type NTRK1 genes, a base is substituted, and as a result, the 595th amino acid residue of wild type TrkA proteins (TrkA isoform2 (RefSeq: NP_002320.2)) or an amino acid residue corresponding thereto (for example, a corresponding amino acid residue in other TrkA isoforms or mutant type TrkA proteins) is converted from glycine into arginine. In addition, the G595R mutation in NTRK1 fusion genes refers to a mutation in which an amino acid residue of the TrkA fusion protein corresponding to the 595th amino acid residue of the wild type TrkA protein (TrkA isoform2 (RefSeq: NP_002320.2)) is converted from glycine into arginine.

The G667C mutation refers to a mutation in which, in wild type or mutant type NTRK1 genes, a base is substituted, and as a result, the 667th amino acid residue of wild type TrkA proteins (TrkA isoform2 (RefSeq: NP_002320.2)) or an amino acid residue corresponding thereto (for example, a corresponding amino acid residue in other TrkA isoforms or mutant type TrkA proteins) is converted from glycine into cysteine. In addition, the G667C mutation in NTRK1 fusion genes refers to a mutation in which an amino acid residue of TrkA fusion proteins corresponding to the 667th amino acid residue of the wild type TrkA proteins (TrkA isoform2 (RefSeq: NP_002320.2)) is converted from glycine into cysteine.

The F589L mutation refers to a mutation in which, in wild type or mutant type NTRK1 genes, a base is substituted, and as a result, the 589th amino acid residue of wild type TrkA proteins (TrkA isoform2 (RefSeq: NP_002320.2)) or an amino acid residue corresponding thereto (for example, a corresponding amino acid residue in other TrkA isoforms or mutant type TrkA proteins) is converted from phenylalanine into leucine. In addition, the F589L mutation in NTRK1 fusion genes refers to a mutation in which an amino acid residue of TrkA fusion proteins corresponding to the 589th amino acid residue of the wild type TrkA proteins (TrkA isoform2 (RefSeq: NP_002320.2)) is converted from phenylalanine into leucine.

The G639R mutation refers to a mutation in which, in wild type or mutant type NTRK2 genes, a base is substituted, and as a result, the 639th amino acid residue of wild type TrkB proteins (TrkB isoform a (RefSeq: NP_006171.2)) or an amino acid residue corresponding thereto (for example, a corresponding amino acid residue in other TrkB isoforms or mutant type TrkB proteins) is converted from glycine into arginine. In addition, the G639R mutation in NTRK2 fusion genes refers to a mutation in which an amino acid residue of the TrkB fusion protein corresponding to the 639th amino acid residue of the wild type TrkB protein (TrkB isoform a (RefSeq: NP_006171.2)) is converted from glycine into arginine. The G639R mutation is a mutation in the TrkB protein corresponding to the above G595R mutation.

The G623R mutations refers to a mutation in which, in wild type or mutant type NTRK3 genes, a base is substituted, and as a result, the 623rd amino acid residue of wild type TrkC proteins (TrkC isoform a (RefSeq: NP_001012338.1) or TrkC isoform b (RefSeq: NP_002521.2)) or an amino acid residue corresponding thereto (for example, a corresponding amino acid residue in other TrkC isoforms or mutant type TrkC proteins) is converted from glycine into arginine. In addition, the G623R mutation in NTRK3 fusion genes refers to a mutation in which an amino acid residue of the TrkC fusion protein corresponding to the 623rd amino acid residue of the wild type TrkC proteins (TrkC isoform a (RefSeq: NP_001012338.1) or TrkC isoform b (RefSeq: NP_002521.2)) is converted from glycine into arginine. The G623R mutation is a mutation in the TrkC protein corresponding to the above G595R mutation.

The G696A mutation refers to a mutation in which, in wild type or mutant type NTRK3 genes, a base is substituted, and as a result, the 696th amino acid residue of wild type TrkC proteins (TrkC isoform a (RefSeq: NP_001012338.1) or TrkC isoform b (RefSeq: NP_002521.2)) or an amino acid residue corresponding thereto (for example, a corresponding amino acid residue in other TrkC isoforms or mutant type TrkC proteins) is converted from glycine into alanine. In addition, the G696A mutation in NTRK3 fusion genes refers to a mutation in which an amino acid residue of the TrkC fusion protein corresponding to the 696th amino acid residue of the wild type TrkC protein (TrkC isoform a (RefSeq: NP_001012338.1) or TrkC isoform b (RefSeq: NP_002521.2)) is converted from glycine into alanine. The G696A mutation is a mutation in the TrkC protein corresponding to the above G667C mutation. The point mutation in NTRK genes (including NTRK fusion genes) which causes Trk inhibitor resistance is not limited to those described above, but includes those found up to now and those to be found in the future.

Examples of other aspects of cancer include Trk inhibitor-resistant cancer. The Trk inhibitor-resistant cancer is cancer in which a therapeutic effect according to a Trk inhibitor is not sufficient. Trk inhibitor-resistant cancers include cancer in which progression or recurrence of symptoms after treatment using a Trk inhibitor has been confirmed, cancer in which a therapeutic effect according to a Trk inhibitor is expected to be insufficient, and cancer in which resistance to the Trk inhibitor after the Trk inhibitor is administered is acquired.

Examples of another aspect of Trk inhibitor-resistant cancer include cancer in which transcription of NTRK genes (including NTRK fusion genes) is enhanced and cancer in which expression of Trk receptors or Trk fusion proteins is enhanced.

Examples of still another aspect of Trk inhibitor-resistant cancer include cancer in which resistance to a Trk inhibitor is acquired by causing a point mutation in NTRK genes. Examples of aspects of point mutations in NTRK genes include a point mutation in kinase domains of NTRK fusion genes, and as a certain aspect, include one or more mutations selected from the group consisting of the above G595R mutations, G667C mutations, F589L, G639R mutations, G623R mutations and G696A mutations.

Examples of still another aspect of Trk inhibitor-resistant cancer include wild type NTRK gene positive cancer.

Here, Trk inhibitor-resistant cancers also include cancer having two or more of the above aspects.

In the present invention, the Trk inhibitor refers to a drug having inhibiting activity toward Trk proteins, and a certain aspect thereof includes 1-{2-[4-(2-amino-5-chloro-3-pyridinyl)phenoxy] -5-pyrimidinyl}-3-[2-(methylsulfonyl)-5-(trifluoromethyl)phenyl]urea, a salt thereof, an N-oxide thereof, a solvate thereof, or a prodrug thereof. In addition, in another aspect, one or more drugs selected from among entrectinib (RXDX-101), LOXO-101, LOXO-195, AZD-7451, TSR-011, crizotinib, altiratinib, ASP-7269, DS-6051b and VM-902 may be exemplified.

In an aspect, a combination of the present invention can be applied to treat metastatic cancer or inhibit metastasis.

In an aspect, the combination of the present invention inhibits recurrence.

[Administration]

In the present invention, a dosage form when the compound represented by General Formula (I), a salt thereof, an N-oxide thereof, a solvate thereof, or a prodrug thereof or one or more drugs selected from the group consisting of an MEK inhibitor, a CDK4/6 inhibitor, an EGFR inhibitor and a JAK1/2 inhibitor are administered in combination (co-administration) may be a form of a combination drug in which both components are combined in one formulation or a dosage form as separate formulations. When administered as separate formulations, they may be administered simultaneously, administered sequentially, or administered at desired time intervals.

In addition, when administered sequentially and administered at desired time intervals,

(1) the compound represented by General Formula (I), a salt thereof, an N-oxide thereof, a solvate thereof, or a prodrug thereof is administered and then one or more drugs selected from the group consisting of an MEK inhibitor, a CDK4/6 inhibitor, an EGFR inhibitor and a JAK1/2 inhibitor may be administered, or
(2) one or more drugs selected from the group consisting of an MEK inhibitor, a CDK4/6 inhibitor, an EGFR inhibitor and a JAK1/2 inhibitor is administered and then the compound represented by General Formula (I), a salt thereof, an N-oxide thereof, a solvate thereof, or a prodrug thereof may be administered. Respective administration methods may be the same or different from each other.

In the present invention, the compound represented by General Formula (I), a salt thereof, an N-oxide thereof, a solvate thereof, or a prodrug thereof is administered in a combination with one or more drugs selected from the group consisting of an MEK inhibitor, a CDK4/6 inhibitor, an EGFR inhibitor and a JAK1/2 inhibitor in order to:

1) perform prevention and/or complementation of the therapeutic effect and/or perform enhancement,
2) reduce the dosage thereof and/or
3) alleviate the side effect thereof.

In the present invention, the dosage of the MEK inhibitor, the CDK4/6 inhibitor, the EGFR inhibitor, the JAK1/2 inhibitor, the compound represented by General Formula (I), a salt thereof, an N-oxide thereof, a solvate thereof, or a prodrug thereof may vary according to the age, weight, symptoms, therapeutic effect, administration methods, treatment period and the like and is generally orally administered once to several times a day (in a certain aspect, once a day, twice a day, three times a day, or the like) within a range of 1 mg to 1,000 mg per dose for an adult. In addition, for an adult, the dosage is parenterally administered within a range of 0.1 mg to 100 mg per dose once to several times a day (in a certain aspect, once a day, twice a day, three times a day, or the like) or intravenously continuously administered within a range of 1 hour to 24 hours a day. As described above, the dosage may vary according to various conditions, thus the sufficient dosage may be of course lower than the amount described above or the amount higher than the above may be required.

In the present invention, the treatment means, for example, exhibiting at least one effect of prolonging progression free survival (PFS), prolonging overall survival (OS), prolonging disease free survival (DFS), prolonging time to progression (TTP), prolonging event-free survival (EFS), prolonging relapse-free survival (RFS), reducing in tumor size, inhibiting (delaying or stopping) tumor growth, inhibiting (delaying or stopping) tumor metastasis, inhibiting (preventing or delaying) recurrence, and alleviating one or more symptoms associated with cancer.

[Other drugs]

Further, the combination of the present invention may be administered in combination with other drugs in order to:

1) perform prevention and/or complementation of the therapeutic effect and/or perform enhancement,
2) reduce the dosage thereof and/or
3) alleviate the side effect thereof.

Examples of other drugs include an alkylating agent, an antimetabolic agent, an anticancer antibiotic, an anticancer plant formulation, a hormone agent, a platinum compound, a topoisomerase inhibitor, a kinase inhibitor, anti-CD20 antibodies, anti-HER2 antibodies, anti-VEGF antibodies, a proteasome inhibitor, an HDAC inhibitor, an immune checkpoint inhibitor (for example, anti-CTLA-4 antibodies, anti-PD-1 antibodies, and anti-PD-LI antibodies), an immunomodulating drug, and other anticancer drugs.

Examples of alkylating agents include cyclophosphamide, ifosfamide, dacarbazine, nimustine hydrochloride, ranimustine, bendamustine, thiotepa, and carboquone.

Examples of antimetabolic agents include methotrexate, pemetrexed, fluorouracil, tegafur, tegafur uracil, tegafur-gimestat-otastat potassium, doxifluridine, capecitabine, cytarabine, gemcitabine hydrochloride, fludarabine, nelarabine, carmofur, and procarbazine hydrochloride.

Examples of anticancer antibiotics include mitomycin C, doxorubicin hydrochloride, aclarubicin hydrochloride, pirarubicin hydrochloride, epirubicin, chromomycin A3, bleomycin, peplomycin sulfate, and therarubicin.

Examples of anticancer plant formulations include irinotecan hydrochloride, etoposide, vincristine sulfate, vinblastine sulfate, vindesine sulfate, vinorelbine tartrate, docetaxel hydrate, eribulin mesylate, and paclitaxel.

Examples of hormone agents include estramustine phosphate sodium, flutamide, bicalutamide, goserelin acetate, leuprorelin acetate, tamoxifen citrate, toremifene citrate, anastrozole, letrozole, exemestane, mepithiostane, medroxyprogesterone acetate, epithiostanol, fosfestrol, fadrozole hydrochloride hydrate, abiraterone, fulvestrant, and aminoglutethimide.

Examples of platinum compounds include carboplatin, cisplatin, nedaplatin, and oxaliplatin.

Examples of topoisomerase inhibitors include topotecan and sobuzoxane.

Examples of kinase inhibitors include lapatinib which is an HER2 inhibitor, imatinib which is a BCR-ABL inhibitor, crizotinib which is an ALK inhibitor, regorafenib and dasatinib which are a multikinase inhibitor, trametinib which is an MEK inhibitor, selumetinib, and binimetinib.

Examples of anti-CD20 antibodies include rituximab, ibritumomab, ibritumomab oxetane, and ocrelizumab.

Examples of anti-HER2 antibodies include trastuzumab, trastuzumab emtansine, and pertuzumab.

Examples of anti-VEGF antibodies include bevacizumab.

Examples of proteasome inhibitors include bortezomib.

Examples of HDAC inhibitors include vorinostat.

Examples of anti-CTLA-4 antibodies include ipilimumab and tremelimumab.

Examples of anti-PD-1 antibodies include nivolumab and pembrolizumab.

Examples of anti-PD-Ll antibodies include atezolizumab and avelumab.

Examples of immunomodulating drugs include thalidomide, lenalidomide, and pomalidomide.

In addition, other drugs include not only those found up to now but also those to be found in the future based on the above mechanism.

[Formulation]

The compound represented by General Formula (I), a salt thereof, an N-oxide thereof, a solvate thereof, or a prodrug thereof, the CDK4/6 inhibitor, the EGFR inhibitor, or the JAK1/2 inhibitor used in the present invention is used as a solid agent for internal use for oral administration, a liquid medicine for internal use, and an injection, an external medicine, a suppository, an ophthalmic solution, an inhalation and the like for parenteral administration.

The oral solid dosage form for internal use may include tablets, pills, capsules, powders, granules and the like. Capsules may include hard capsules and soft capsules. Tablets may include sublingual tablets, oral patches, orally disintegrating tablets and the like.

In the solid dosage form for internal use, one or more active substances per se may be formulated or may be formulated after mixing thereof with a excipient (lactose, mannitol, glucose, microcrystalline cellulose, starch and the like), a binder (hydroxypropylcellulose, polyvinylpyrrolidone, magnesium aluminate metasilicate and the like), a disintegrant (calcium cellulose glycolate and the like), a lubricant (magnesium stearate and the like), a stabilizer, a solution adjuvant (glutamic acid, aspartic acid and the like) according to conventional methods. The solid dosage form may be optionally coated with a coating agent (sucrose, gelatin, hydroxypropylcellulose, hydroxypropyl methylcellulose phthalate and the like) and may be coated with two or more layers. The solid dosage form may further encompass capsules of an absorbable substance such as gelatin.

The internal liquid medicine may include pharmaceutically acceptable waters, suspensions, emulsions, syrups, elixirs and the like. In the liquid medicine, one or more active substances are dissolved, suspended or emulsified in a diluent of general use (purified water, ethanol or a mixed solution thereof). The liquid medicine may further contain a wetting agent, a suspending agent, an emulsifying agent, a sweetening agent, a flavouring agent, an aroma, a preservative, a buffering agent and the like.

The dosage form of the external medicine for parenteral administration may include, for example, ointments, gels, creams, cataplasms, plasters and pressure sensitive adhesives, liniments, atomized agents, inhalations, sprays, aerosols, ophthalmic solutions, nasal solutions and the like. The dosage forms contain one or more active substances and may be prepared according to well known methods or formulations which are generally used.

Atomized agents, inhalations and sprays may contain, in addition to a diluent which is generally used, a stabilizer such as sodium hydrogen sulfite and a buffering agent that confers isotonicity, e.g., sodium chloride, sodium citrate or an isotonicity agent such as citric acid. Methods for producing sprays are specifically described in, for example, U.S. Patent No. 2,868,691 and U.S. Patent No. 3,095,355.

The injection for parenteral administration may encompass injections in the form of solution, suspension, emulsion and solid that is dissolved or suspended in a solvent upon use. The injection may be used by dissolving, suspending or emulsifying one or more active substances in a solvent. The solvent may be, for example, distilled water for injection, saline, vegetable oil, propylene glycol, polyethylene glycol, alcohols such as ethanol and combinations thereof. The injection may further contain a stabilizer, a solution adjuvant (glutamic acid, aspartic acid, Polysorbate 80 (registered trademark) and the like), a suspending agent, an emulsifying agent, a soothing agent, a buffering agent, a preservative and the like. The injection may be produced by sterilization in the final step or through aseptic technique. Aseptic solid agents, e.g., lyophilized products may be manufactured and dissolved in sterilized or aseptic distilled water or other solvents for injection before use.

Other compositions for parenteral administration may include suppositories for rectal administration and pessaries for vaginal administration which contain one or more active substances and are formulated according to conventional methods.

Unless otherwise defined, all technical and scientific terms and abbreviations used herein have the same meanings as are usually understood by a person skilled in the art to which the present invention pertains.

[Toxicity]

The combination of the present invention has sufficiently low toxicity and can be used as safely as a medicament.

Examples

Hereinafter, the present invention will be described in detail with reference to examples and the present invention is not limited thereto. The compound represented by General Formula (I), a salt thereof, an N-oxide thereof, a solvate thereof, a prodrug thereof, or a crystal thereof can be produced according to the method described in WO 2014/129431 or WO 2016/027754, a known method, or a method equivalent thereto.

Pharmacological Experiment Examples: Pharmacological Experiment Example 1: Measurement of TrkA kinase-inhibiting activity using human TrkA-expressing cells

TrkA kinase-inhibiting activity in cell systems was measured using CHO-K1 cells exressing human TrkA and NFAT-bla (CellSenser™ TrkA-NFAT-*bla* CHO-K1 cells, Invitrogen).

On the day before the assay, CellSenser™ TrkA-NFAT-*bla* CHO-K1 cells were suspended in an assay medium (Opti-MEM1 Reduced Serum Medium (Invitrogen) containing 0.5 vol% dialysed fetal bovine serum (Invitrogen), 0.1 mM nonessential amino acids (Invitrogen), 1 mM sodium pyruvate (Invitrogen) and antibiotics (100 U/mL penicillin and 100 μg/mL streptomycin (Invitrogen))) and plated at a density of $2.4 \times 10^4$ cells/40 μL/well in a 96-well clear bottom plate (Corning, Catalogue No.: 3882). In some wells were added only the assay medium at 40 μL/well (Cell-free).

On the day of the assay, 10 mM of the present compound (DMSO solution) was distributed in a 96-well plate (Costar, Catalogue No.: 3363) and serially diluted with DMSO with the geometrical ratio of 3. The serial dilutions were diluted with the assay medium to 100-fold to prepare a solution of the present compound with a 10-fold concentration (DMSO concentration: 1 vol%). To the plate where cells were plated was added the present compound at 5 μL/well and the plate was incubated in a $CO_2$ incubator with 5% $CO_2$, 95% air at 37°C for 30 minutes. For a control and a blank, the assay medium containing 1 vol% DMSO was added at 5 μL/well in place of the solution of the present compound. Subsequently the assay medium containing NGF (Mouse 2.5s, Natural, Invitrogen) was added to the plate at 5 μL/well (final concentration of NGF: 50 ng/ml) and the plate was incubated in a $CO_2$ incubator with 5% $CO_2$, 95% air at 37°C for 5 hours. For the blank group, the assay medium was added in place of NGF at 5 μL/well. A reporter assay detection reagent (10 μL/well) was added to the plate which was then incubated in the dark at room temperature for 120 minutes.

The reporter assay detection reagent was prepared from LiveBLAzer™-FRET B/G Loading Kit (Invitrogen) . On the Analyst GT (Molecular Devices Japan, K.K.) the wells were irradiated with excitation light at 405 nm and the fluorescence intensities at 460 nm and 530 nm were measured. The time resolved fluorescence resonance energy transfer (TR-FRET) ratio of each well was calculated according to the following mathematical formula:

[Math. 1]

$$\text{TR-FRET ratio} = (A_{460X} - A_{460F})/(A_{530X} - A_{530F})$$

wherein,

$A_{460X}$ is the fluorescence intensity at 460 nm of the present compound, control or blank;
$A_{460F}$ is the fluorescence intensity at 460 nm of the Cell-free; $A_{530X}$ is the fluorescence intensity at 530 nm of the present compound, control or blank; and
$A_{530F}$ is the fluorescence intensity at 530 nm of the Cell-free.

The TR-FRET inhibition rate (%) of the present compound was calculated according to the following mathematical formula:

[Math. 2]

$$\text{Inhibition rate (\%)} = \{1 - (A_X - A_B)/(A_C - A_B)\} \times 100$$

wherein,

$A_X$ is the TR-FRET ratio when the present compound is added;
$A_B$ is the TR-FRET of the blank; and
$A_C$ is the TR-FRET of the control.

The $IC_{50}$ value by the present compound was calculated from the inhibition curve based on the inhibition rate of the present compound at respective concentrations.

As a result, it was found that the present compounds had $IC_{50}$ values of 0.5 μM or lower and had TrkA-inhibiting activity. $IC_{50}$ values of some of the present compounds are shown in the following tables.

[Table 1]

| Compound name | TrkA inhibiting activity (IC50; μM) | Compound name | TrkA inhibiting activity (IC50; μM) |
|---|---|---|---|
| 1-(2-(1H-pyrazol-1-yl)-5-(trifluoromethyl)phenyl)-3-(2-(4-(2-amino-5-chloropyridin-3-yl)phenoxy) pyrimidin-5-yl)urea | 0.001 | 1-(2-(4-(2-amino-5-chloropyridin-3-yl) phenoxy)pyrimidin-5-yl)-3-(2-chloro-4-(trifluoromethyl)phenyl)urea | 0.002 |
| 1-(2-(4-(2-amino-5-chloropyridin-3-yl) phenoxy)pyrimidin-5-yl)-3-(2-(4-methyl-1H-1,2,3-triazol-1-yl)-5-(trifluoromethyl)phenyl)urea | 0.001 | 1-(2-{4-[2-amino-5-(trifluoromethyl)-3-pyridinyl]phenoxy}-5-pyrimidinyl)-3-{5-(trifluoromethyl)-2-[3-(trifluoromethyl)-1H-pyrazol-1-yl]phenyl}urea | 0.004 |
| 1-(2-(4-(2-amino-5-chloropyridin-3-yl) phenoxy)pyrimidin-5-yl)-3-(5-(trifluoromethyl)-2-(3-(trifluoromethyl)-1H-pyrazol-1-yl) phenyl)urea | 0.002 | 1-{2-[4-(2-amino-5-chloro-3-pyridinyl) phenoxy]-5-pyrimidinyl}-3-[4-(trifluoromethyl)-2-biphenylyl]urea | 0.004 |
| 1-(2-(4-(2-amino-5-chloropyridin-3-yl) phenoxy)pyrimidin-5-yl)-3-(2-chloro-5-(trifluoromethyl)phenyl)urea | 0.001 | 1-{2-[4-(2-amino-5-fluoro-3-pyridinyl) phenoxy]-5-pyrimidinyl}-3-[4-(trifluoromethyl)-2-biphenylyl]urea | 0.002 |
| 1-(2-(1H-pyrazol-1-yl)-5-(trifluoromethyl)phenyl-3-(6-(4-(2-amino-5-chloropyridin-3-yl)phenoxy) pyridin-3-yl)urea | 0.003 | 1-{2-[4-(2-amino-5-chloro-3-pyridinyl) phenoxy]-5-pyrimidinyl}-3-[2-(4-chloro-1H-pyrazol-1-yl)-5-(trifluoromethyl)phenyl]urea | 0.002 |
| 1-(2-(1H-1,2,3-triazol-1-yl)-5-(trifluoromethyl)phenyl-3-(6-(4-(2-amino-5-chloropyridin-3-yl)phenoxy) pyridin-3-yl)urea | 0.001 | 1-{2-[4-(2-amino-5-chloro-3-pyridinyl) phenoxy]-5-pyrimidinyl} -3-{5-chloro-2-[3-(trifluoromethyl)-1H-pyrazol-1-yl] phenyl}urea | 0.002 |

(continued)

| Compound name | TrkA inhibiting activity (IC50; μM) | Compound name | TrkA inhibiting activity (IC50; μM) |
|---|---|---|---|
| 1-(6-(4-(2-amino-5-chloropyridin-3-yl) phenoxy)pyridin-3-yl)-3-(2-(pyridin-3-yl)-5-(trifluoromethyl)phenyl)urea | 0.001 | 1-{2-[4-(2-amino-5-chloro-3-pyridinyl) phenoxy]-5-pyrimidinyl}-3-[2,4-bis (trifluoromethyl)phenyl]urea | 0.002 |
| 1-(2-(4-(2-amino-5-chloropyridin-3-yl) phenoxy)pyrimidin-5-yl)-3-(2-(1-methyl-1H-pyrazol-5-yl)-5-(trifluoromethyl)phenyl)urea | 0.002 | 1-(2-{4-[2-amino-5-(trifluoromethyl)-3-pyridinyl]phenoxy}-5-pyrimidinyl)-3-[2-(4-chloro-1H-pyrazol-1-yl)-5-(trifluoromethyl) phenyl]urea | 0.001 |
| 1-(2-(1H-1,2,3-triazol-1-yl)-5-(trifluoromethyl)phenyl)-3-(2-(4-(2-amino-5-fluoropyridin-3-yl)phenoxy) pyrimidin-5-yl)urea | 0.001 | 1-{2-[4-(2-amino-5-fluoro-3-pyridinyl) phenoxyl-5-pyrimidinyl}-3-[2-(methylsulfonyl)-5-(trifluoromethyl) phenyl] urea | 0.0004 |
| 1-(2-(4-(2-amino-5-fluoropyridin-3-yl) phenoxy)pyrimidin-5-yl)-3-(2-(pyridin-3-yl)-5-(trifluoromethyl)phenyl)urea | 0.001 | 1-(2-{4-[2-amino-5-(trifluoromethyl)-3-pyridinyl]phenoxy}-5-pyrimidinyl)-3-[2-(methylsulfonyl)-5-(trifluoromethyl) phenyl]urea | 0.0008 |
| 1-(2-(1H-pyrazol-1-yl)-4-(trifluoromethyl)phenyl)-3-(2-(4-(2-amino-5-chloropyridin-3-yl)phenoxy) pyrimidin-5-yl)urea | 0.001 | 1-{2-[4-(2-amino-5-chloro-3-pyridinyl) phenoxy]-5-pyrimidinyl}-3-[2-(methylsulfonyl)-5-(trifluoromethyl) phenyl]urea | 0.0005 |
| 1-(2-(4-(2-amino-5-chloropyridin-3-yl) phenoxy)pyrimidin-5-yl)-3-(2-fluoro-4-(trifluoromethyl)phenyl)urea | 0.003 | 2-{[(2-{4-[2-amino-5-(trifluoromethyl)-3-pyridinyl]phenoxy}-5-pyrimidinyl) carbamoyl]amino}-N,N-dimethyl-4-(trifluoromethyl)benzenesulfonamide | 0.004 |

[Table 2]

| Compound name | TrkA inhibiting activity (IC50; μM) | Compound name | TrkA inhibiting activity (IC50; μM) |
|---|---|---|---|
| 1-(2-(4-(5-(azetidin-1-yl)pyrazolo[1,5-a] pyrimidin-3-yl)phenoxy)pyrimidin-5-yl)-3-(2-(pyridin-3-yl)-5-(trifluoromethyl)phenyl) urea | 0.001 | 1-(2-(1H-pyrazol-1-yl)-5-(trifluoromethyl)phenyl)-3-(2-(4-(6-methoxyimidazo[1,2-b] pyridazin-3-yl)phenoxy)pyrimidin-5-yl)urea | 0.001 |
| 1-(2-(4-(5-(azetidin-1-yl)pyrazolo[1,5-a] pyrimidin-3-yl)phenoxy)pyrimidin-5-yl)-3-(2-(1-methyl-1H-pyrazol-5-yl)-5-(trifluoromethyl) phenyl)urea | 0.001 | 1-{2-[4-(6-ethoxyimidazo[1,2-b] pyridazin-3-yl)phenoxy]-5-pyrimidinyl}-3-[2-(3-pyridinyl)-5-(trifluoromethyl)phenyl]urea | 0.001 |
| 1-(2-(4-(5-methoxypyrazolo[1,5-a]pyrimidin-3-yl)phenoxy)pyrimidin-5-yl)-3-(2-(pyridin-3-yl)-5-(trifluoromethyl)phenyl)urea | 0.001 | 1-(2-(1H-pyrazol-1-yl)-5-(trifluoromethyl)phenyl)-3-(2-(4-(imidazo[1,2-a]pyridin-3-yl) phenoxy)pyrimidin-5-yl)urea | 0.001 |

(continued)

| Compound name | TrkA inhibiting activity (IC50; μM) | Compound name | TrkA inhibiting activity (IC50; μM) |
|---|---|---|---|
| 1-(2-(4-(pyrazolo[1,5-a]pyrimidin-3-yl)phenoxy) pyrimidin-5-yl)-3-(2-(pyridin-3-yl)-5-(trifluoromethyl)phenyl)urea | 0.001 | 1-(2-(4-(1H-pyrrolo[2,3-b]pyridin-5-yl)phenoxy)pyrimidin-5-yl)-3-(2-(pyridin-3-yl)-5-(trifluoromethyl)phenyl)urea | 0.002 |
| 1-(2-(4-[5-(dimethylamino)pyrazolo[1,5-a] pyrimidin-3-yl]phenoxy}-5-pyrimidinyl)-3-[2-(3-pyridinyl)-5-(trifluoromethyl)phenyl]urea | 0.001 | 1-(2-(4-(imidazo[1,2-a]pyrazin-3-yl) phenoxy)pyrimidin-5-yl)-3-(2-(pyridin-3-yl)-5-(trifluoromethyl)phenyl)urea | 0.001 |
| 1-(2-{4-[5-(dimethylamino)pyrazolo[1,5-a] pyrimidin-3-yl]phenoxy}-5-pyrimidinyl)-3-[2-(1-methyl-1H-pyrazol-5-yl)-5-(trifluoromethyl) phenyl]urea | 0.001 | 1-(2-(4-(pyrazolo[1,5-a]pyridin-3-yl) phenoxy)pyrimidin-5-yl)-3-(2-(pyridin-3-yl)-5-(trifluoromethyl)phenyl)urea | 0.001 |
| 1-[2-[4-(5-methylpyrazolo[1,5-a]pyrimidin-3-yl) phenoxy]-5-pyrimidinyl}-3-[2-(3-pyridinyl)-5-(trifluoromethyl)phenyl]urea | 0.0007 | 1-(2-(4-(1H-pyrazolo[3,4-b]pyridin-5-yl)phenoxy)pyrimidin-5-yl)-3-(2-(pyridin-3-yl)-5-(trifluoromethyl)phenyl)urea | 0.001 |
| 1-(2-(4-[5-(ethylamino)pyrazolo[1,5-a] pyrimidin-3-yl]phenoxy}-5-pyrimidinyl)-3-[3'-methyl-4-(trifluoromethyl)-2-biphenylyl]urea | 0.004 | | |
| 1-(2-14-[5-(dimethylamino)pyrazolo[1,5-a] pyrimidin-3-yl]phenoxy}-5-pyrimidinyl)-3-[4-(trifluoromethyl)-2-biphenylyl]urea | 0.002 | | |
| 1-(2-{4-[5-(dimethylamino)pyrazolo[1,5-a] pyrimidin-3-yl]phenoxy}-5-pyrimidinyl)-3-[3'-methyl-4-(trifluoromethyl)-2-biphenylyl]urea | 0.003 | | |
| 1-(2-{4-[5-(dimethylamino)pyrazolo[1,5-a] pyrimidin-3-yl]phenoxy}-5-pyrimidinyl)-3-[2'-methyl-4-(trifluoromethyl)-2-biphenylyl]urea | 0.004 | | |
| 1-(2-(4-(6-methoxyimidazo[1,2-b]pyridazin-3-yl)phenoxy)pyrimidin-5-yl)-3-(2-(pyridin-3-yl)-5-(trifluoromethyl)phenyl)urea | 0.001 | | |

Pharmacological Experiment Example 2: Enzyme-inhibiting activity test of kinases other than Trk (selectivity experiment)

A test substance (the present compound) was dissolved in dimethylsulfoxide to adjust a 100-fold concentration of the test concentration, 3 μM. The solution was further diluted to 25-fold with an assay buffer (20 mM HEPES, 0.01 vol% Triton X-100, 2 mM DTT, pH 7.5) to obtain a test substance solution. In a similar manner a positive control substance solution was prepared with a positive control substance.

A 4-fold concentration solution (5 μL) of the test substance adjusted with the assay buffer, 5 μL of a 4-fold concentration solution of substrate/ATP/metal (Mg) and 10 μL of a 2-fold concentration solution of kinase were mixed in a well of a polypropylene 384-well plate and allowed to react at room temperature for 1 hour. The reaction was terminated by adding 60 μL of a Termination Buffer (QuickScout Screening Assist MSA; Carna Biosciences). The substrate peptide and the phosphorylated peptide in the reaction solution were separated and quantified. The kinase reaction was assessed from the product ratio (P/(P+S)) calculated from the height (S) of the peak of the substrate peptide and the height (P) of the peak of the phosphorylated peptide. Other kinases used in the kinase selectivity experiments were, for example, b-Raf and KDR. The following table indicates substrates, substrate concentrations, ATP concentrations and positive control substances used in respective kinase enzyme inhibition activity tests.

[Table 3]

| Kinase | Substrate | | ATP (μM) | Positive control |
|---|---|---|---|---|
| | Name | (nM) | | |
| b-Raf | MAP2K1 | 1 | 1,000 | ZM336372 |
| KDR | CSKtide | 1,000 | 75 | Staurosporine |

The inhibition rate was calculated from the average signal intensity of the test wells containing respective test substances provided that the average signal intensity of control wells each containing all reaction components was 0% inhibition and the average signal intensity of background wells (without addition of the enzyme) was 100% inhibition. As a result, the present compounds at a concentration of 3 μM had the inhibition rates of kinases as shown in the following table.

[Table 4]

| Compound name | Inhibition rate (%) | | Compound name | Inhibition rate (%) | |
|---|---|---|---|---|---|
| | b-Raf | KDR | | b-Raf | KDR |
| 1-(2-(1H-pyrazol-1-yl)-5-(trifluoromethyl)phenyl)-3-(2-(4-(2-amino-5-chloropyridin-3-yl)phenoxy)pyrimidin-5-yl)urea | 40 | 0 | 1-{2-[4-(2-amino-5-fluoro-3-pyridinyl)phenoxyl-5-pyrimidinyl}-3-[2-(methylsulfonyl)-5-(trifluoromethyl)phenyl]urea | 22 | 2 |
| 1-(2-(4-(2-amino-5-chloropyridin-3-yl)phenoxy)pyrimidin-5-yl)-3-(2-(4-methyl-1H-1,2,3-triazol-1-yl)-5-(trifluoromethyl)phenyl)urea | 6.5 | 0 | 1-(2-(4-[2-amino-5-(trifluoromethyl)-3-pyridinyl]phenoxy}-5-pyrimidinyl)-3-[2-(methylsulfonyl)-5-(trifluoromethyl)phenyl]urea | 23 | 8 |
| 1-(2-(4-(2-amino-5-chloropyridin-3-yl)phenoxy)pyrimidin-5-yl)-3-(5-(trifluoromethyl)-2-(3-(trifluoromethyl)-1H-pyrazol-1-yl)phenyl)urea | 21 | 0 | 1-{2-[4-(2-amino-5-chloro-3-pyridinyl)phenoxyl-5-pyrimidinyl}-3-[2-(methylsulfonyl)-5-(trifluoromethyl)phenyl]urea | 53 | 18 |
| 1-(2-(4-(2-amino-5-chloropyridin-3-yl)phenoxy)pyrimidin-5-yl)-3-(2-(1-methyl-1H-pyrazol-5-yl)-5-(trifluoromethyl)phenyl)urea | 33 | 7.5 | 2-{[(2-(4-[2-amino-5-(trifluoromethyl)-3-pyridinyl]phenoxy}-5-pyrimidinyl)carbamoyl]amino}-N,N-dimethyl-4-(trifluoromethyl)benzenesulfonamide | 26 | 0 |
| 1-(2-(1H-1,2,3-triazol-1-yl)-5-(trifluoromethyl)phenyl)-3-(2-(4-(2-amino-5-fluoropyridin-3-yl)phenoxy)pyrimidin-5-yl)urea | 13 | 0 | 1-(2-(4-(5-(azetidin-1-yl)pyrazolo[1,5-a]pyrimidin-3-yl)phenoxy)pyrimidin-5-yl)-3-(2-(pyridin-3-yl)-5-(trifluoromethyl)phenyl)urea | 36 | 1.5 |
| 1-(2-(4-(2-amino-5-fluoropyridin-3-yl)phenoxy)pyrimidin-5-yl)-3-(2-(pyridin-3-yl)-5-(trifluoromethyl)phenyl)urea | 45 | 1.7 | 1-(2-(4-(5-(azetidin-1-yl)pyrazolo[1,5-a]pyrimidin-3-yl)phenoxy)pyrimidin-5-yl)-3-(2-(1-methyl-1H-pyrazol-5-yl)-5-(trifluoromethyl)phenyl)urea | 30 | 1 |
| 1-(2-(1H-pyrazol-1-yl)-4-(trifluoromethyl)phenyl)-3-(2-(4-(2-amino-5-chloropyridin-3-yl)phenoxy)pyrimidin-5-yl)urea | 0 | 0 | 1-(2-{4-[5-(dimethylamino)pyrazolo[1,5-a]pyrimidin-3-yl]phenoxy}-5-pyrimidinyl)-3-[2-(3-pyridinyl)-5-(trifluoromethyl)phenyl]urea | 50 | 16 |
| 1-(2-(4-(2-amino-5-chloropyridin-3-yl)phenoxy)pyrimidin-5-yl)-3-(2-chloro-4-(trifluoromethyl)phenyl)urea | 37 | 0 | 1-(2-{4-[5-(dimethylamino)pyrazolo[1,5-a]pyrimidin-3-yl]phenoxy}-5-pyrimidinyl)-3-[2-(1-methyl-1H-pyrazol-5-yl)-5-(trifluoromethyl)phenyl]urea | 49 | 9 |

(continued)

| Compound name | Inhibition rate (%) | | Compound name | Inhibition rate (%) | |
|---|---|---|---|---|---|
| | b-Raf | KDR | | b-Raf | KDR |
| 1-(2-{4-[2-amino-5-(trifluoromethyl)-3-pyridinyl]phenoxyl-5-pyrimidinyl)-3-(5-l (trifluoromethyl)-2-[3-(trifluoromethyl)-1H-pyrazol-1-yl]phenyl}urea | 20 | 0 | 1-{2-[4-(5-methylpyrazolo[1,5-a] pyrimidin-3-yl)phenoxyl-5-pyrimidinyl}-3-[2-(3-pyridinyl)-5-(trifluoromethyl) phenyl]urea | 58 | 19 |
| 1-{2-[4-(2-amino-5-chloro-3-pyridinyl) phenoxy]-5-pyrimidinyl}-3-[4-(trifluoromethyl)-2-biphenylyl]urea | 30 | 0 | 1-(2-{4-[5-(ethylamino)pyrazolo[1,5-alpyrimidin-3-yl]phenoxyl-5-pyrimidinyl)-3-[3'-methyl-4-(trifluoromethyl)-2-biphenylyl]urea | 19 | 0 |
| 1-{2-[4-(2-amino-5-fluoro-3-pyridinyl) phenoxy]-5-pyrimidinyl}-3-[4-(trifluoromethyl)-2-biphenylyl]urea | 30 | 0 | 1-(2-{4-[5-(dimethylamino)pyrazolo [1,5-a]pyrimidin-3-yl]phenoxy}-5-pyrimidinyl)-3-[4-(trifluoromethyl)-2-biphenylyl]urea | 19 | 0 |
| 1-{2-[4-(2-amino-5-chloro-3-pyridinyl) phenoxyl-5-pyrimidinyl}-3-[2-(4-chloro-1H-pyrazol-1-yl)-5-(trifluoromethyl) phenyl]urea | 43 | 0 | 1-(2-{4-[5-(dimethylamino)pyrazolo [1,5-a]pyrimidin-3-yl]phenoxy}-5-pyrimidinyl)-3-[3'-methyl-4-(trifluoromethyl)-2-biphenylyl]urea | 21 | 0 |
| 1-{2-[4-(2-amino-5-chloro-3-pyridinyl) phenoxyl-5-pyrimidinyl}-3-(5-chloro-2-[3-(trifluoromethyl)-1H-pyrazol-1-yl] phenyl}urea | 40 | 0 | 1-(2-{4-[5-(dimethylamino)pyrazolo [1,5-a]pyrimidin-3-yl]phenoxy}-5-pyrimidinyl)-3-[2'-methyl-4-(trifluoromethyl)-2-biphenylyl]urea | 41 | 0 |
| 1-{2-[4-(2-amino-5-chloro-3-pyridinyl) phenoxy]-5-pyrimidinyl}-3-[2,4-bis (trifluoromethyl)phenyl]urea | 32 | 0 | 1-{2-[4-(6-ethoxyimidazo[1,2-b] pyridazin-3-yl)phenoxy]-5-pyrimidinyl}-3-[2-(3-pyridinyl)-5-(trifluoromethyl) phenyl]urea | 56 | 19 |
| 1-(2-{4-[2-amino-5-(trifluoromethyl)-3-pyridinyl]phenoxy}-5-pyrimidinyl)-3-[2-(4-chloro-1H-pyrazol-1-yl)-5-(trifluoromethyl)phenyl]urea | 34 | 0 | | | |

From this result, it is demonstrated that the present compounds show low inhibition of kinases other than TrkA, e.g., b-Raf and KDR, while exhibit strong inhibition of TrkA. In other words, the present compounds have TrkA inhibition as strong as $IC_{50}$ of 0.5 μM or less according to the result from Pharmacological Example 1, while the present compounds inhibit kinases other than TrkA only at 0% to about 58% even at the concentration of 3 μM according to the result from Pharmacological Example 2. Thus it is demonstrated that the present compounds have high selectivity towards TrkA inhibition and have excellent kinase selectivity.

Pharmacological Experiment Example 3: Growth inhibition test in TPM3-NTRK1 positive KM12 cell lines

(1) Experiment method

Using KM12 as TPM3-NTRK1-positive human colon cancer cell lines, an effect when 1-{2-[4-(2-amino-5-chloro-3-pyridinyl)phenoxy]-5-pyrimidinyl}-3-[2-(methylsulfonyl)-5-(trifluoromethyl)phenyl]urea (hereinafter sometimes referred to as a compound A) and trametinib, selumetinib, palbociclib, afatinib or erlotinib were combined was evaluated. Cells were subcultured using a DMEM containing a 10 vol% inactivated fetal bovine serum (FBS) and 1 vol% penicillin-streptomycin liquid as a culture medium before they underwent the experiment.

One day before the compound was treated, cells were floated using 0.25% Trypsin-EDTA, and the cells were collected into a centrifuge tube from a culture flask. At room temperature, 180 g of the cells were centrifuged for 3 minutes, and the cell sediment was then suspended in a DMEM culture medium. A part of the cell suspension was

collected, the number of cells was counted, and the cells were then suspended in the DMEM culture medium at a cell density of $3.75 \times 10^4$ cells/mL to prepare a cell suspension. The cell suspension was inoculated at 80 μL/well in a 96-well tissue culture plate (Asahi Glass Co., Ltd.), and left for 24 hours under 37°C, 5% $CO_2$, and 95% air conditions.

On the day when the compound was treated, 10 mM of each compound (DMSO solution) was serially diluted with DMSO to prepare a diluted solution. The diluted solution of each compound and DMSO were diluted 100-fold in a culture medium to prepare a 10-fold concentration of the treatment solution of each compound and the medium solution. The compound A solution or medium solution prepared above was added at 10 μL/well to wells of a 96-well tissue culture plate in which cells were stationary-cultured for 24 hours. Then, in the same manner, the trametinib, selumetinib, palbociclib, afatinib or erlotinib solution or the medium solution was added at 10 μL/well to wells of the 96-well tissue culture plate. After the compound was added, stationary-culture was performed for 72 hours under 37°C, 5% $CO_2$, and 95% air conditions.

After the stationary culture was completed, a luminescent signal (relative luminescent unit, RLU) of each well was measured by a microplate reader using a CellTiter-Glo Luminescent Cell Viability Assay kit (Promega, G7571). An average value of RLUs of 3 wells of a medium group (a group treated with a medium solution having a compound concentration of zero (0)) was calculated, and a growth inhibition rate in each well was calculated by the following mathematical formula.

[Math. 3]

$$\text{Growth inhibition rate (\%)} = \{1 - (\text{RLU of each well}/(\text{average value of RLUs of medium group}))\} \times 100$$

In order to analyze the combined effect, using a fluorescence intensity, the effective dose $(ED)_{50}$, $ED_{75}$, $ED_{90}$ and $ED_{95}$ were calculated, and a combination index (CI) described in Advances in enzyme regulation, vol. 22, 1984, pp. 27-55 was calculated for analysis. CI is a method that is generally used to determine the strength of the combined effect. According to the score of CI, it was determined that a synergistic effect was exhibited in the case of CI<1, an additive effect was exhibited in the case of CI=1, and an antagonistic effect was exhibited in the case of CI>1.

(2) Results

In TPM3-NTRK1 positive KM12, the compound A and any of various kinase inhibitors were used in combination, and as a result, as shown in Fig. 1 and Fig. 4, compared to the single treatment, a stronger growth inhibitory effect was observed. In addition, as shown in the following table, when the compound A and trametinib, selumetinib, palbociclib or afatinib were used in combination, the CI value was less than 1 in all cases, and a synergistic effect according to the combination was observed. Based on the above results, it was confirmed that, when the Trk inhibitor such as the compound A and any of various kinase inhibitors were used in combination, a strong antitumor effect was exhibited.

[Table 5]

| | Trametinib | Selumetinib | Palbociclib | Afatinib |
|---|---|---|---|---|
| CI (median value) | 0.705 | 0.674 | 0.660 | 0.861 |

Pharmacological Experiment Example 4: Growth inhibition experiment in ETV6-NTRK3 positive IMS-M2 cell lines

(1) Experiment method

Using IMS-M2 as ETV6-NTRK3 positive human leukemia cell lines, an effect when the compound A and trametinib, selumetinib, palbociclib, afatinib or ruxolitinib were combined was evaluated. Cells were subcultured using an RPMI culture medium containing a 10 vol% FBS and 1 vol% penicillin-streptomycin liquid before they underwent the experiment.

On the day when the compound was treated, cells were collected from a culture flask into a centrifuge tube. At room temperature, 300 g of cells were centrifuged for 3 minutes, and the cell sediment was then suspended in the RPMI culture medium. A part of the cell suspension was collected, the number of cells was counted, and the cells were

then suspended in the RPMI culture medium at a cell density of 6.25×$10^4$ cells/mL to prepare a cell suspension. The cell suspension was inoculated at 80 μL/well in a 96-well tissue culture plate (Asahi Glass Co., Ltd.) and left under 37°C, 5% $CO_2$, and 95% air conditions.

10 mM of the compound A, palbociclib, afatinib and ruxolitinib (DMSO solution) were serially diluted with DMSO to prepare a diluted solution of each compound. The diluted solution of each compound and DMSO were diluted 100-fold in a culture medium to prepare a 10-fold concentration of the treatment solution of each compound and the medium solution.

The compound A solution or medium solution prepared above was added at 10 μL/well to wells of a 96-well tissue culture plate. Then, in the same manner, the palbociclib, afatinib, ruxolitinib solution or medium solution was added at 10 μL/well to wells of the 96-well tissue culture plate. After the compound was added, stationary culture was performed under 37°C, 5% $CO_2$, and 95% air conditions for 72 hours. After the stationary culture was completed, the growth inhibition rate and the CI value were calculated in the same methods as in Pharmacological Experiment Example 3.

(2) Results

In ETV6-NTRK3 positive IMS-M2 cell lines, the compound A and any of various kinase inhibitors were used in combination, and as a result, as shown in Fig. 2 and Fig. 5, compared to the single treatment, a stronger growth inhibitory effect was observed. In addition, as shown in the following table, when the compound A and trametinib, selumetinib, palbociclib, afatinib or ruxolitinib were used in combination, the CI value was less than 1 in all cases, and a synergistic effect according to the combination was observed. Based on the above results, it was confirmed that, when the Trk inhibitor such as the compound A and any of various kinase inhibitors were used in combination, a strong antitumor effect was exhibited.

[Table 6]

| | Trametinib | Selumetinib | Palbociclib | Afatinib | Ruxolitinib |
|---|---|---|---|---|---|
| CI (median value) | 0.652 | 0.549 | 0.719 | 0.869 | 0.862 |

Pharmacological Experiment Example 5: Growth inhibition test in compound A resistant KM12 cell lines

(1) Establishment of compound A resistant KM12 cell lines (KM12-AR)

Using KM12 as TPM3-NTRK1-positive human colon cancer cell lines, a strain resistant to the compound A was established. A DMEM (Life technologies, product number: 11965) containing a 10 vol% FBS and 1 vol% penicillin-streptomycin liquid was prepared as a culture medium. The thawed KM12 was suspended in the culture medium. At room temperature,180 g was centrifuged for 3 minutes, the supernatant was removed by suction, and the cell sediment was then re-suspended in the culture medium, and a total amount of 50 mL was inoculated in a 225 $cm^2$ flask (Asahi Glass Co., Ltd.). 50 μL of the compound A that was diluted with DMSO to reach 3 μM was added to the DMEM culture medium (3 nM as a final concentration) and left under 37°C, 5% $CO_2$, and 95% air conditions.

Then, every two or three days, the culture medium was replaced or subcultured. In the subculture, cells were floated using 0.25% Trypsin-EDTA (Invitrogen), the cells were collected in a centrifuge tube, and at room temperature, 180 g of KM12 was then centrifuged for 3 minutes. The cell sediment was suspended in the DMEM culture medium, a part of the cell suspension was then collected, and the number of viable cells was counted. A part of the cell suspension was inoculated in a 225 $cm^2$ flask, a total amount of the culture medium was adjusted to 50 mL and 50 μL of the compound A that was diluted with DMSO to reach 3 μM was then added (3 nM as a final concentration), and left under 37°C, 5% $CO_2$, and 95% air conditions. In this method, KM12 was subcultured while gradually increasing the concentration of the compound A to the final concentration of 6 nM. KM12 that had been subcultured ten times in the DMEM culture medium containing 6 nM of the compound A was used for the growth inhibition test as KM12-AR.

Here, when the NTRK1 gene sequence of KM12-AR was checked by a Sanger sequencing method (Thermo Fisher Scientific, 3500 Genetic Analyzer), no point mutation was observed in the TrkA kinase domain.

(2) Growth inhibition experiment for KM12-AR

Using KM12-AR, an effect when the compound A and trametinib, selumetinib, palbociclib, gefitinib, erlotinib, afatinib, lapatinib, neratinib or canertinib were combined was evaluated.

Cells were subcultured in a DMEM culture medium containing the compound A having a final concentration of 6 nM before they underwent the experiment. 48 hours before the compound was treated, the culture medium was

replaced with a DMEM culture medium containing no compound A and left for 24 hours under 37°C, 5% $CO_2$, and 95% air conditions. Cells were floated using 0.25% Trypsin-EDTA and the cells were collected into a centrifuge tube from a culture flask. At room temperature, 180 g of the cells were centrifuged for 3 minutes, and the cell sediment was then suspended in 10 mL of the DMEM culture medium. A part of the cell suspension was collected, the number of cells was counted, and the cells were then suspended in the DMEM culture medium at a cell density of $5\times10^4$ cells/mL to prepare a cell suspension. The cell suspension was inoculated at 50 μL/well in a 96-well tissue culture plate (Asahi Glass Co., Ltd.) and left under 37°C, 5% $CO_2$, and 95% air conditions for 24 hours.

On the day when the compound was treated, 10 mM of each compound (DMSO solution) was serially diluted with DMSO to prepare a diluted solution. The diluted solution of each compound and DMSO were diluted 50-fold in a culture medium, and additionally diluted 10-fold in a DMEM culture medium to prepare a 4-fold concentration of the treatment solution of each compound and the medium solution. The compound A solution or medium solution prepared above was added at 25μL/well to wells of a 96-well tissue culture plate in which cells were stationary-cultured for 24 hours. Then, in the same manner, the trametinib, selumetinib, palbociclib, gefitinib, erlotinib, afatinib, lapatinib, neratinib, canertinib solution or medium solution was added at 25 μL/well to wells of the 96-well tissue culture plate. After the compound was added, the growth inhibition rate was calculated in the same method as in Pharmacological Experiment Example 3.

(3) Results

In KM12-AR, the compound A and any of various kinase inhibitors were used in combination, and as a result, as shown in Fig. 3, Fig. 6 and Fig. 7, compared to the single treatment, a stronger growth inhibitory effect was observed. In particular, when the compound A was treated alone and/or when any of various kinase inhibitors was treated alone, no growth inhibitory effect was observed. However, when the compound A and any of various kinase inhibitors were treated in combination, it was confirmed that a growth inhibitory effect was exhibited.

Based on the above results, it was confirmed that, when the Trk inhibitor such as the compound A and any of various kinase inhibitors were used in combination, a strong antitumor effect was exhibited. In KM12-AR, it was inferred that the compound A resistance was acquired by inducing activation of alternative pathways such as MEK, EGFR, CDK, and JAK in the presence of the compound A.

[Formulation Examples]

Formulation Example 1

The following components are mixed according to a conventional method and then compressed to give 10,000 tablets containing 10 mg of the active ingredient per tablet.

- 1-(2-(4-(2-amino-5-chloropyridin-3-yl)phenoxy)pyrimidin-5-yl)-3-(2-(4-methyl-1*H*-1,2,3-triazol-1-yl)-5-(trifluoromethyl)phenyl)urea ... 100 g
- Calcium carboxymethylcellulose (disintegrating agent) ... 20 g
- Magnesium stearate (lubricant) ... 10 g
- Microcrystalline cellulose ... 870 g

Formulation Example 2

The following components are mixed according to a conventional method, filtered through a dust filter, distributed to ampoules at 5 ml and thermally sterilized in an autoclave to obtain 10,000 ampoules containing 20 mg of the active ingredient per ampoule.

- 1-(2-(4-(2-amino-5-chloropyridin-3-yl)phenoxy)pyrimidin-5-yl)-3-(2-(4-methyl-1*H*-1,2,3-triazol-1-yl)-5-(trifluoromethyl)phenyl)urea ... 200 g
- Mannitol ... 20 g
- Distilled water ... 50 L

[Industrial Applicability]

The combination of the compound represented by General Formula (I), a salt thereof, an N-oxide thereof, a solvate thereof, or a prodrug thereof, and one or more drugs selected from the group consisting of an MEK inhibitor, a CDK4/6 inhibitor, an EGFR inhibitor and a JAK1/2 inhibitor can be beneficially used to treat cancer.

## Claims

1. An agent for treating cancer, the agent being used such that the agent is administered in combination with one or more drugs selected from the group consisting of an MEK inhibitor, a CDK4/6 inhibitor, an EGFR inhibitor and a JAK1/2 inhibitor, the agent containing a compound represented by General Formula (I) :

[C1]

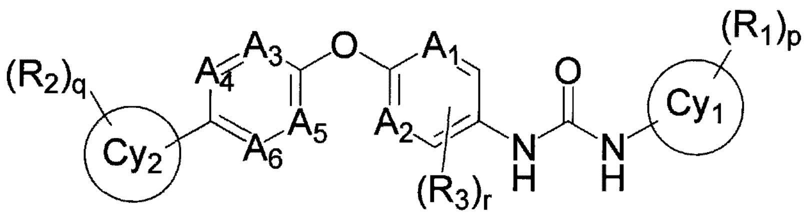

(I)

wherein,

a ring $Cy_1$ represents a C3-10 monocyclic carbocycle or bicyclic carbocycle or a 4- to 10-membered monocyclic heterocycle or bicyclic heterocycle,
a ring $Cy_2$ represents a 4- to 10-membered monocyclic heterocycle or bicyclic heterocycle,
$R_1$ represents

(1) a halogen,
(2) a C1-6 alkyl group, C2-6 alkenyl group or C2-6 alkynyl group optionally substituted with a substituent selected from the group consisting of (i) a halogen and (ii) a hydroxy group,
(3) a C5-6 monocyclic carbocycle optionally substituted with one or two $R_5$ groups,
(4) a 5- to 6-membered monocyclic heterocycle optionally substituted with one or two $R_5$ groups,
(5) $-S(O)_{m1}-R_6$,
(6) $-SO_2NR_7R_8$,
(7) $-C(O)OR_9$,
(8) $-NR_{10}C(O)R_{11}$,
(9) $-C(O)NR_{12}R_{13}$,
(10) $-OR_{14}$,
(11) $-NR_{15}R_{16}$,
(12) a cyano group, or
(13) a nitro group,

$R_5$ represents

(1) a halogen,
(2) $-S(O)_{m2}-R_{17}$,
(3) $-SO_2NR_{18}R_{19}$,
(4) $-C(O)OR_{20}$,
(5) $-NR_{21}C(O)R_{22}$,
(6) $-C(O)NR_{23}R_{24}$,
(7) $-OR_{25}$,
(8) $-NR_{26}R_{27}$,
(9) a cyano group,
(10) a nitro group, or
(11) a C1-3 alkyl group optionally substituted with a substituent selected from the group consisting of (i) a halogen, (ii) a hydroxy group and (iii) an oxo group,

when two $R_5$ groups are present, the $R_5$ groups may be independently the same or different,
when, further, two $R_5$ groups are each independently a C1-3 alkyl group or a hydroxy group and the $R_5$ groups are attached to carbon atoms adjacent to each other on the C5-6 monocyclic carbocycle or the 5- to 6-membered monocyclic heterocycle, the $R_5$ groups may together form a ring,
$R_6$ to $R_{27}$ each independently represent (1) a hydrogen atom or (2) a C1-6 alkyl group optionally substituted

with (i) a halogen or (ii) a hydroxy group,
when $R_{18}$ and $R_{19}$ are each independently a C1-6 alkyl group, $R_{18}$ and $R_{19}$ groups may together form a ring,
$R_2$ represents

(1) a halogen,
(2) a C1-6 alkyl group optionally substituted with (i) a halogen or (ii) a hydroxy group,
(3) a C3-6 cycloalkyl group optionally substituted with (i) a halogen or (ii) a hydroxy group,
(4) a C1-6 alkoxy group optionally substituted with a halogen,
(5) $-NR_{28}R_{29}$,
(6) a 3- to 7-membered monocyclic heterocycle, or
(7) -O-(3- to 7-membered monocyclic heterocycle),

$R_{28}$ and $R_{29}$ each independently represent (1) a hydrogen atom or (2) a C1-6 alkyl group optionally substituted with (i) a halogen or (ii) a hydroxy group,
$A_1$ and $A_2$ each independently represent $=CR_3$- or =N-,
$A_3$, $A_4$, $A_5$ and $A_6$ each independently represent $=CR_4$- or =N-,
$R_3$ and $R_4$ each independently represent a hydrogen atom or a halogen,
m1 represents an integer of 0 to 2,
m2 represents an integer of 0 to 2,
p represents an integer of 0 to 7,
q represents an integer of 0 to 7,
r represents an integer of 0 to 2,
provided that, when $R_1$, $R_2$, $R_3$ and $R_4$ each independently occur twice or more, $R_1$, $R_2$, $R_3$ and $R_4$ may each independently be the same or different,
a salt thereof, an N-oxide thereof, a solvate thereof, or a prodrug thereof.

**2.** An agent for treating cancer, the agent being used such that the agent is administered in combination with a compound represented by General Formula (I):

[C2]

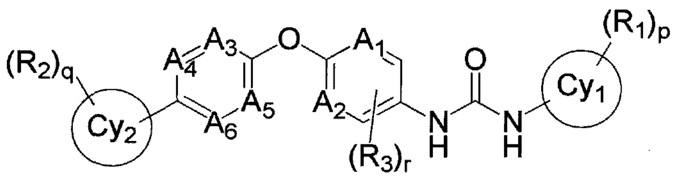

(I)

wherein all symbols have the same meanings as in claim 1, a salt thereof, an N-oxide thereof, a solvate thereof, or a prodrug thereof, the agent containing one or more drugs selected from the group consisting of an MEK inhibitor, a CDK4/6 inhibitor, an EGFR inhibitor and a JAK1/2 inhibitor.

**3.** An agent for treating cancer, the agent containing:

one or more drugs selected from the group consisting of an MEK inhibitor, a CDK4/6 inhibitor, an EGFR inhibitor and a JAK1/2 inhibitor; and
a compound represented by General Formula (I):

[C3]

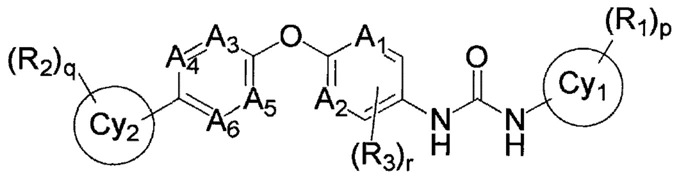

(I)

wherein all symbols have the same meanings as in claim 1, a salt thereof, an N-oxide thereof, a solvate thereof,

or a prodrug thereof.

**4.** The agent according to any one of claims 1 to 3, wherein the compound represented by General Formula (I), a salt thereof, an N-oxide thereof, a solvate thereof, or a prodrug thereof is

(1) 1-(2-(1*H*-pyrazol-1-yl)-5-(trifluoromethyl)phenyl)-3-(2-(4-(2-amino-5-chloropyridin-3-yl)phenoxy)pyrimidin-5-yl)urea,
(2) 1-(2-(4-(2-amino-5-chloropyridin-3-yl)phenoxy)pyrimidin-5-yl)-3-(2-(4-methyl-1*H*-1,2,3-triazol-1-yl)-5-(trifluoromethyl)phenyl)urea,
(3) 1-(2-(4-(2-amino-5-chloropyridin-3-yl)phenoxy)pyrimidin-5-yl)-3-(5-(trifluoromethyl)-2-(3-(trifluoromethyl)-1*H*-pyrazol-1-yl)phenyl)urea,
(4) 1-(2-(4-(2-amino-5-chloropyridin-3-yl)phenoxy)pyrimidin-5-yl)-3-(2-chloro-5-(trifluoromethyl)phenyl)urea,
(5) 1-(2-(1*H*-pyrazol-1-yl)-5-(trifluoromethyl)phenyl-3-(6-(4-(2-amino-5-chloropyridin-3-yl)phenoxy)pyridin-3-yl)urea,
(6) 1-(2-(1*H*-1,2,3-triazol-1-yl)-5-(trifluoromethyl)phenyl-3-(6-(4-(2-amino-5-chloropyridin-3-yl)phenoxy)pyridin-3-yl)urea,
(7) 1-(6-(4-(2-amino-5-chloropyridin-3-yl)phenoxy)pyridin-3-yl)-3-(2-(pyridin-3-yl)-5-(trifluoromethyl)phenyl)urea,
(8) 1-(2-(4-(2-amino-5-chloropyridin-3-yl)phenoxy)pyrimidin-5-yl)-3-(2-(1-methyl-1*H*-pyrazol-5-yl)-5-(trifluoromethyl)phenyl)urea,
(9) 1-(2-(1*H*-1,2,3-triazol-1-yl)-5-(trifluoromethyl)phenyl)-3-(2-(4-(2-amino-5-fluoropyridin-3-yl)phenoxy)pyrimidin-5-yl)urea,
(10) 1-(2-(4-(2-amino-5-fluoropyridin-3-yl)phenoxy)pyrimidin-5-yl)-3-(2-(pyridin-3-yl)-5-(trifluoromethyl)phenyl)urea,
(11) 1-(2-(1*H*-pyrazol-1-yl)-4-(trifluoromethyl)phenyl)-3-(2-(4-(2-amino-5-chloropyridin-3-yl)phenoxy)pyrimidin-5-yl)urea,
(12) 1-(2-(4-(2-amino-5-chloropyridin-3-yl)phenoxy)pyrimidin-5-yl)-3-(2-fluoro-4-(trifluoromethyl)phenyl)urea,
(13) 1-(2-(4-(2-amino-5-chloropyridin-3-yl)phenoxy)pyrimidin-5-yl)-3-(2-chloro-4-(trifluoromethyl)phenyl)urea,
(14) 1-(2-{4-[2-amino-5-(trifluoromethyl)-3-pyridinyl]phenoxy}-5-pyrimidinyl)-3-{5-(trifluoromethyl)-2-[3-(trifluoromethyl)-1*H*-pyrazol-1-yl]phenyl}urea,
(15) 1-{2-[4-(2-amino-5-chloro-3-pyridinyl)phenoxyl-5-pyrimidinyl}-3-[4-(trifluoromethyl)-2-biphenylyl]urea,
(16) 1-{2-[4-(2-amino-5-fluoro-3-pyridinyl)phenoxy]-5-pyrimidinyl}-3-[4-(trifluoromethyl)-2-biphenylyl]urea,
(17) 1-{2-[4-(2-amino-5-chloro-3-pyridinyl)phenoxy]-5-pyrimidinyl}-3-[2-(4-chloro-1*H*-pyrazol-1-yl)-5-(trifluoromethyl)phenyl]urea,
(18) 1-{2-[4-(2-amino-5-chloro-3-pyridinyl)phenoxyl-5-pyrimidinyl}-3-{5-chloro-2-[3-(trifluoromethyl)-1*H*-pyrazol-1-yl]phenyl}urea,
(19) 1-{2-[4-(2-amino-5-chloro-3-pyridinyl)phenoxy]-5-pyrimidinyl}-3-[2,4-bis(trifluoromethyl)phenyl]urea,
(20) 1-(2-{4-[2-amino-5-(trifluoromethyl)-3-pyridinyl]phenoxy}-5-pyrimidinyl)-3-[2-(4-chloro-1*H*-pyrazol-1-yl)-5-(trifluoromethyl)phenyl]urea,
(21) 1-{2-[4-(2-amino-5-fluoro-3-pyridinyl)phenoxy]-5-pyrimidinyl}-3-[2-(methylsulfonyl)-5-(trifluoromethyl)phenyl]urea,
(22) 1-(2-{4-[2-amino-5-(trifluoromethyl)-3-pyridinyl]phenoxy}-5-pyrimidinyl)-3-[2-(methylsulfonyl)-5-(trifluoromethyl)phenyl]urea,
(23) 1-{2-[4-(2-amino-5-chloro-3-pyridinyl)phenoxy]-5-pyrimidinyl}-3-[2-(methylsulfonyl)-5-(trifluoromethyl)phenyl]urea,
(24) 2-{[(2-{4-[2-amino-5-(trifluoromethyl)-3-pyridinyl]phenoxy}-5-pyrimidinyl)carbamoyl]amino}-N,N-dimethyl-4-(trifluoromethyl)benzenesulfonamide,
(25) 1-(2-(4-(5-(azetidin-1-yl)pyrazolo[1,5-a]pyrimidin-3-yl)phenoxy)pyrimidin-5-yl)-3-(2-(pyridin-3-yl)-5-(trifluoromethyl)phenyl)urea,
(26) 1-(2-(4-(5-(azetidin-1-yl)pyrazolo[1,5-a]pyrimidin-3-yl)phenoxy)pyrimidin-5-yl)-3-(2-(1-methyl-1*H*-pyrazol-5-yl)-5-(trifluoromethyl)phenyl)urea,
(27) 1-(2-(4-(5-methoxypyrazolo[1,5-a]pyrimidin-3-yl)phenoxy)pyrimidin-5-yl)-3-(2-(pyridin-3-yl)-5-(trifluoromethyl)phenyl)urea,
(28) 1-(2-(4-(pyrazolo[1,5-a]pyrimidin-3-yl)phenoxy)pyrimidin-5-yl)-3-(2-(pyridin-3-yl)-5-(trifluoromethyl)phenyl)urea,
(29) 1-(2-{4-[5-(dimethylamino)pyrazolo[1,5-a]pyrimidin-3-yl]phenoxy}-5-pyrimidinyl)-3-[2-(3-pyridinyl)-5-(trifluoromethyl)phenyl]urea,

(30) 1-(2-{4-[5-(dimethylamino)pyrazolo[1,5-a]pyrimidin-3-yl]phenoxy}-5-pyrimidinyl)-3-[2-(1-methyl-1*H*-pyrazol-5-yl)-5-(trifluoromethyl)phenyl]urea,
(31) ]1-{2-[4-(5-methylpyrazolo[1,5-a]pyrimidin-3-yl)phenoxy-5-pyrimidinyl}-3-[2-(3-pyridinyl)-5-(trifluoromethyl)phenyl]urea,
(32) 1-(2-{4-[5-(ethylamino)pyrazolo[1,5-a]pyrimidin-3-yl]phenoxy}-5-pyrimidinyl)-3-[3'-methyl-4-(trifluoromethyl)-2-biphenylyl]urea,
(33) 1-(2-14-[5-(dimethylamino)pyrazolo[1,5-a]pyrimidin-3-yl]phenoxy}-5-pyrimidinyl)-3-[4-(trifluoromethyl)-2-biphenylyl]urea,
(34) 1-(2-{4-[5-(dimethylamino)pyrazolo[1,5-a]pyrimidin-3-yl]phenoxy}-5-pyrimidinyl)-3-[3'-methyl-4-(trifluoromethyl)-2-biphenylyl]urea, or
(35) 1-(2-(4-[5-(dimethylamino)pyrazolo[1,5-a]pyrimidin-3-yl]phenoxy}-5-pyrimidinyl)-3-[2'-methyl-4-(trifluoromethyl)-2-biphenylyl]urea, a salt thereof, an N-oxide thereof, a solvate thereof, or a prodrug thereof.

**5.** The agent according to any one of claims 1 to 4,
wherein the MEK inhibitor is trametinib or selumetinib.

**6.** The agent according to any one of claims 1 to 4,
wherein the CDK4/6 inhibitor is palbociclib.

**7.** The agent according to any one of claims 1 to 4,
wherein the EGFR inhibitor is one or more drugs selected from the group consisting of gefitinib, erlotinib, neratinib, canertinib and afatinib.

**8.** The agent according to any one of claims 1 to 4,
wherein the JAK1/2 inhibitor is ruxolitinib.

**9.** The agent according to any one of claims 1 to 8,
wherein the cancer is NTRK gene positive cancer.

**10.** The agent according to claim 9,
wherein the NTRK gene positive cancer is NTRK fusion gene positive cancer.

**11.** The agent according to any one of claims 1 to 10,
wherein the cancer is Trk inhibitor-resistant cancer.

**12.** The agent according to claim 11,
wherein the Trk inhibitor-resistant cancer is cancer resistant to 1-{2-[4-(2-amino-5-chloro-3-pyridinyl)phenoxy]-5-pyrimidinyl}-3-[2-(methylsulfonyl)-5-(trifluoromethyl)phenyl]urea, a salt thereof, an N-oxide thereof, a solvate thereof, or a prodrug thereof.

**13.** The agent according to claim 11,
wherein the Trk inhibitor-resistant cancer is cancer resistant to one or more drugs selected from the group consisting of entrectinib, LOXO-101, LOXO-195, AZD-7451, TSR-011, crizotinib, altiratinib, ASP-7269, DS-6051b and VM-902.

**14.** The agent according to any one of claims 1 to 13,
wherein the cancer is lung cancer, colon cancer, intrahepatic cholangiocarcinoma, thyroid cancer, skin cancer, breast cancer, head and neck cancer, renal cancer, sarcoma, brain tumor, salivary gland tumor or blood cancer.

**15.** A cancer treatment method comprising:

administering an effective amount of the compound represented by General Formula (I), a salt thereof, an N-oxide thereof, a solvate thereof or a prodrug thereof according to claim 1 to a patient,
wherein the treatment further comprises administering an effective amount of one or more drugs selected from the group consisting of an MEK inhibitor, a CDK4/6 inhibitor, an EGFR inhibitor and a JAK1/2 inhibitor.

**16.** The compound represented by General Formula (I), a salt thereof, an N-oxide thereof, a solvate thereof or a prodrug thereof according to claim 1 which is used to treat cancer in combination with one or more drugs selected from the group consisting of an MEK inhibitor, a CDK4/6 inhibitor, an EGFR inhibitor and a JAK1/2 inhibitor.

**17.** Use of the compound represented by General Formula (I), a salt thereof, an N-oxide thereof, a solvate thereof or a prodrug thereof according to claim 1 for producing a cancer therapeutic agent administered in combination with one or more drugs selected from the group consisting of an MEK inhibitor, a CDK4/6 inhibitor, an EGFR inhibitor and a JAK1/2 inhibitor.

FIG. 1

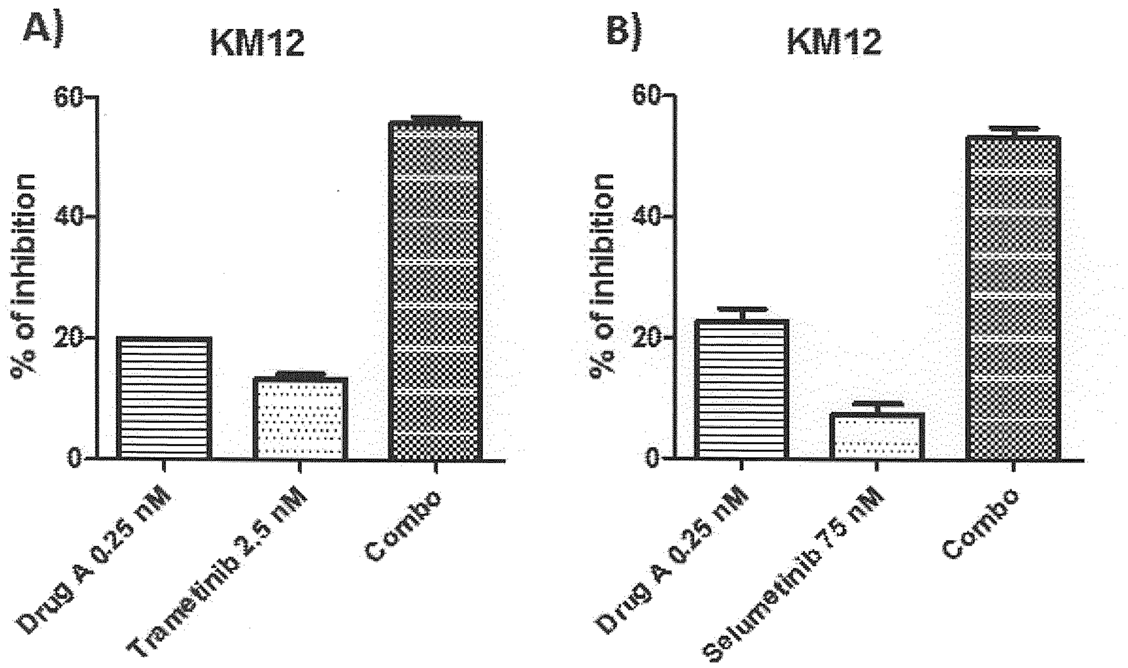
A)
KM12
% of inhibition
60
40
20
0
Drug A 0.25 nM
Trametinib 2.5 nM
Combo
B)
KM12
% of inhibition
60
40
20
0
Drug A 0.25 nM
Selumetinib 75 nM
Combo

FIG. 2

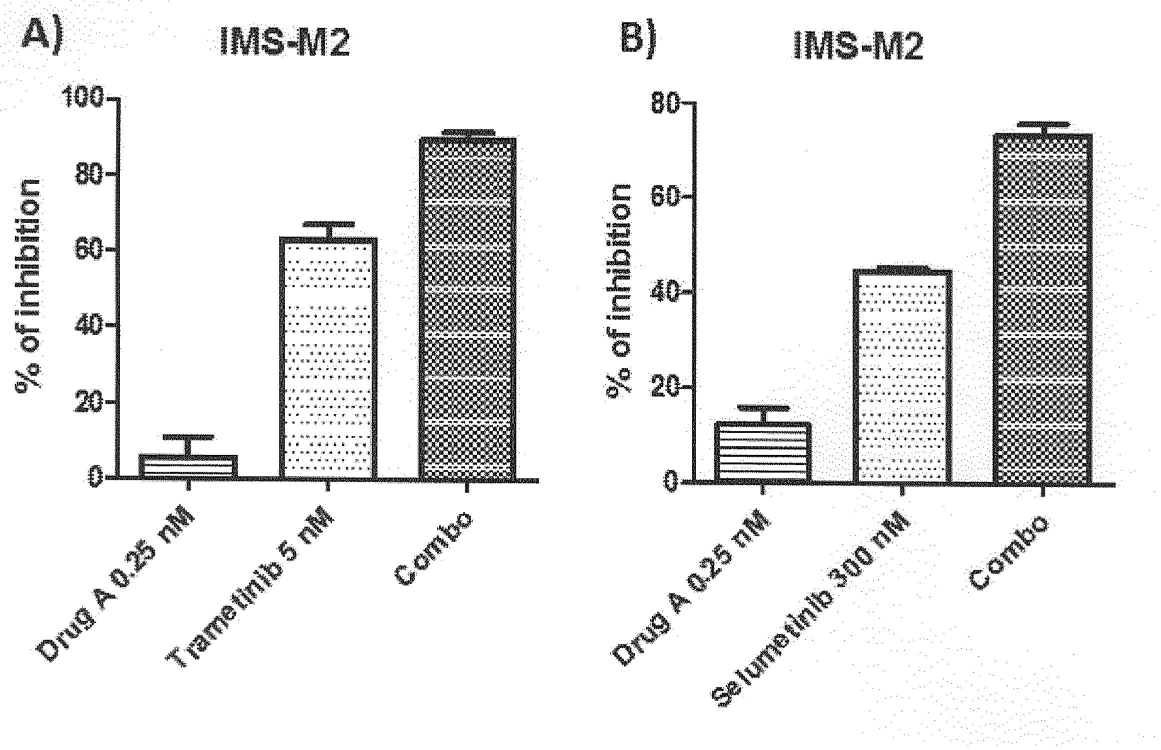
A)
IMS-M2
% of inhibition
100
80
60
40
20
0
Drug A 0.25 nM
Trametinib 5 nM
Combo
B)
IMS-M2
% of inhibition
80
60
40
20
0
Drug A 0.25 nM
Selumetinib 300 nM
Combo

FIG. 3

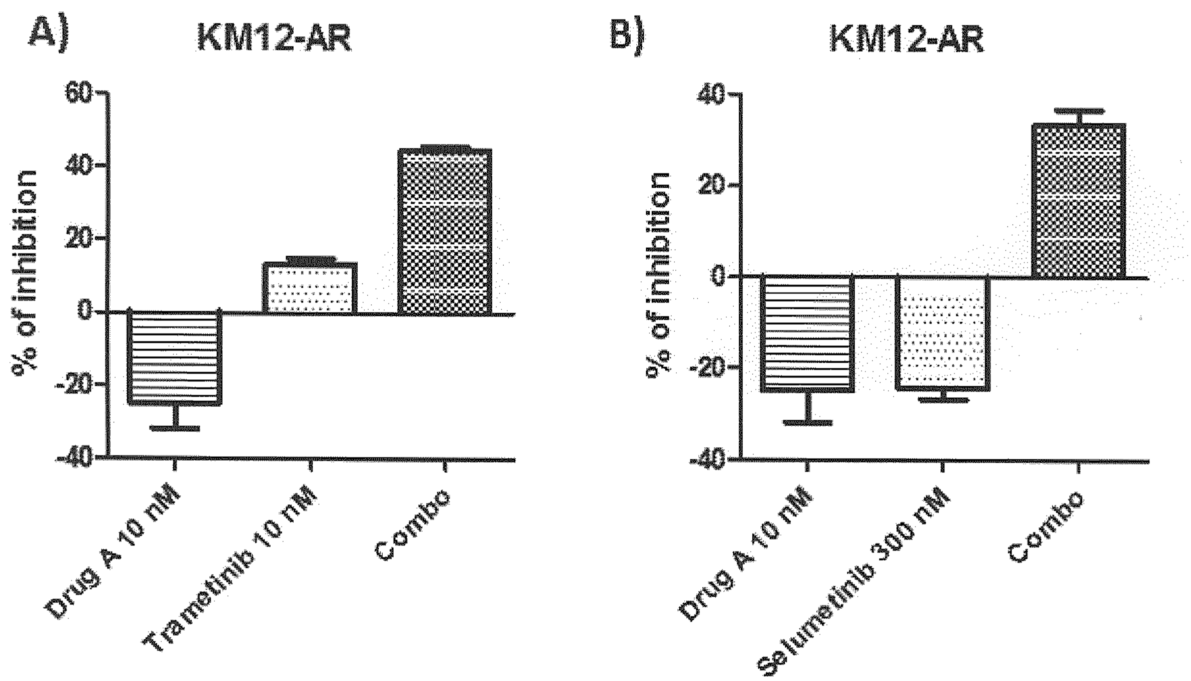
A)
KM12-AR
% of inhibition
60
40
20
0
-20
-40
Drug A 10 nM
Trametinib 10 nM
Combo
B)
KM12-AR
% of inhibition
40
20
0
-20
-40
Drug A 10 nM
Selumetinib 300 nM
Combo

FIG. 4

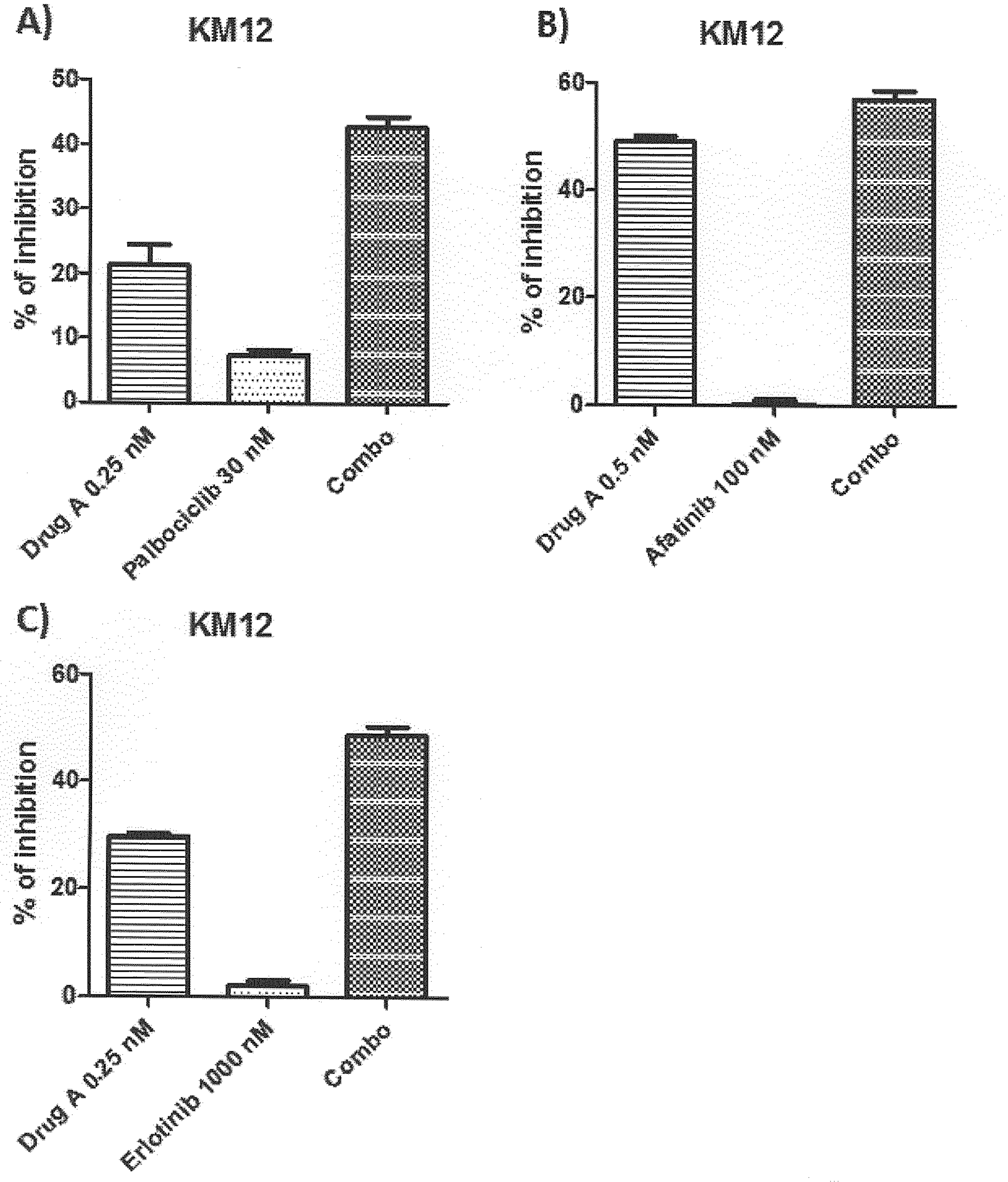
A)
KM12
% of inhibition
0
10
20
30
40
50
Drug A 0.25 nM
Palbociclib 30 nM
Combo
B)
KM12
% of inhibition
0
20
40
60
Drug A 0.5 nM
Afatinib 100 nM
Combo
C)
KM12
% of inhibition
0
20
40
60
Drug A 0.25 nM
Erlotinib 1000 nM
Combo

FIG. 5

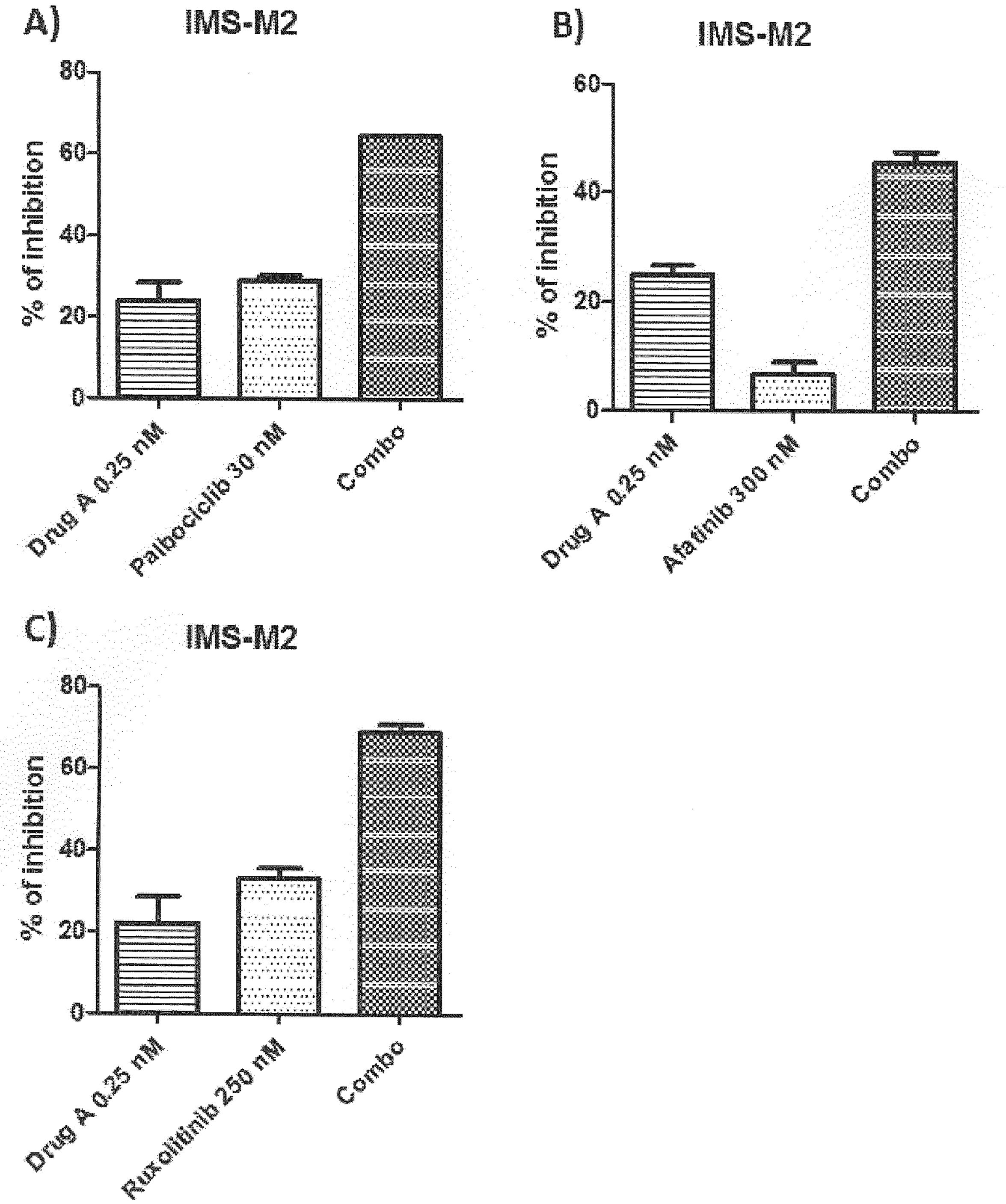
A)
IMS-M2
% of inhibition
0
20
40
60
80
Drug A 0.25 nM
Palbociclib 30 nM
Combo
B)
IMS-M2
% of inhibition
0
20
40
60
Drug A 0.25 nM
Afatinib 300 nM
Combo
C)
IMS-M2
% of inhibition
0
20
40
60
80
Drug A 0.25 nM
Ruxolitinib 250 nM
Combo

FIG. 6

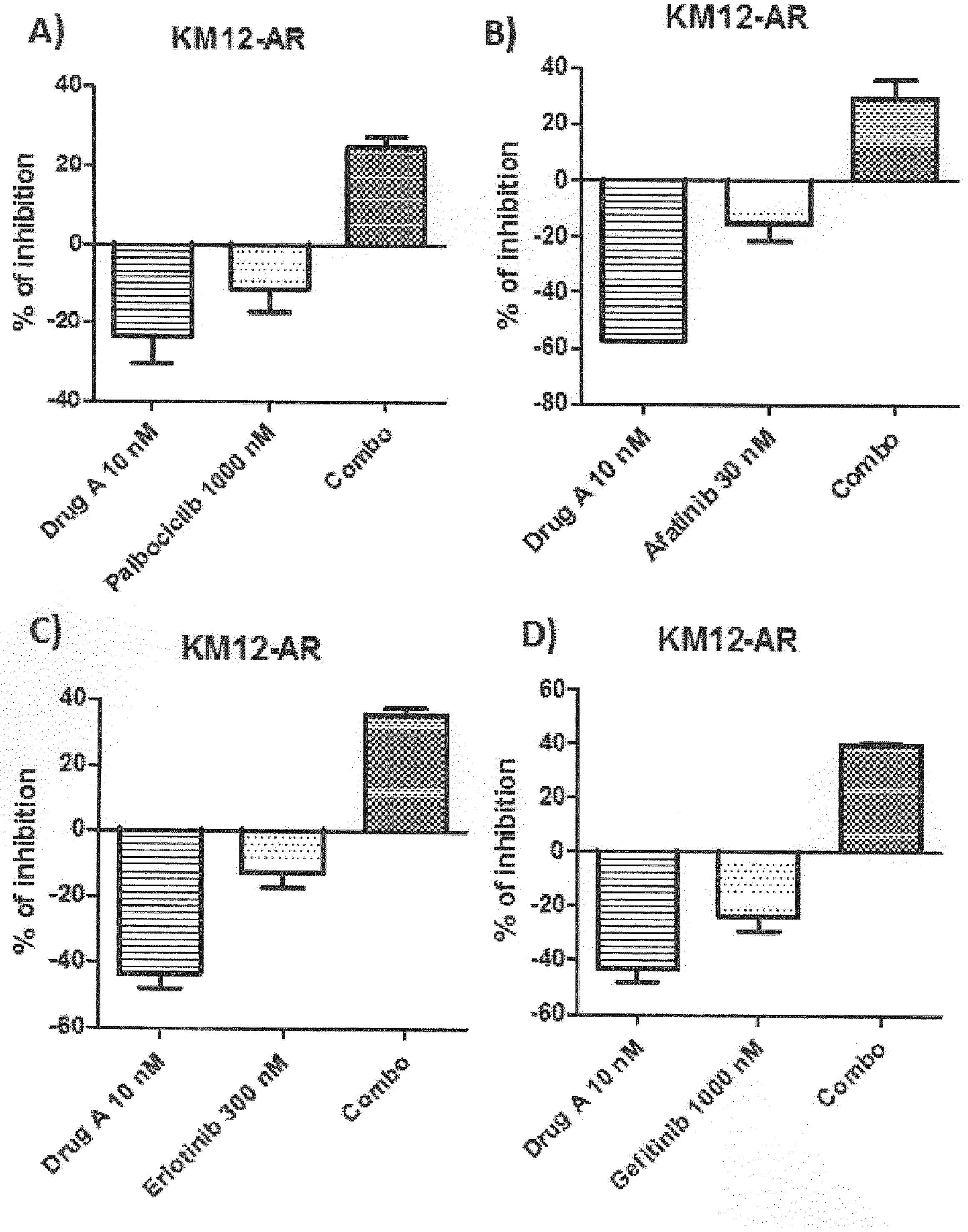
A)
KM12-AR
% of inhibition
40
20
0
-20
-40
Drug A 10 nM
Palbociclib 1000 nM
Combo
B)
KM12-AR
% of inhibition
40
20
0
-20
-40
-60
-80
Drug A 10 nM
Afatinib 30 nM
Combo
C)
KM12-AR
% of inhibition
40
20
0
-20
-40
-60
Drug A 10 nM
Erlotinib 300 nM
Combo
D)
KM12-AR
% of inhibition
60
40
20
0
-20
-40
-60
Drug A 10 nM
Gefitinib 1000 nM
Combo

FIG. 7

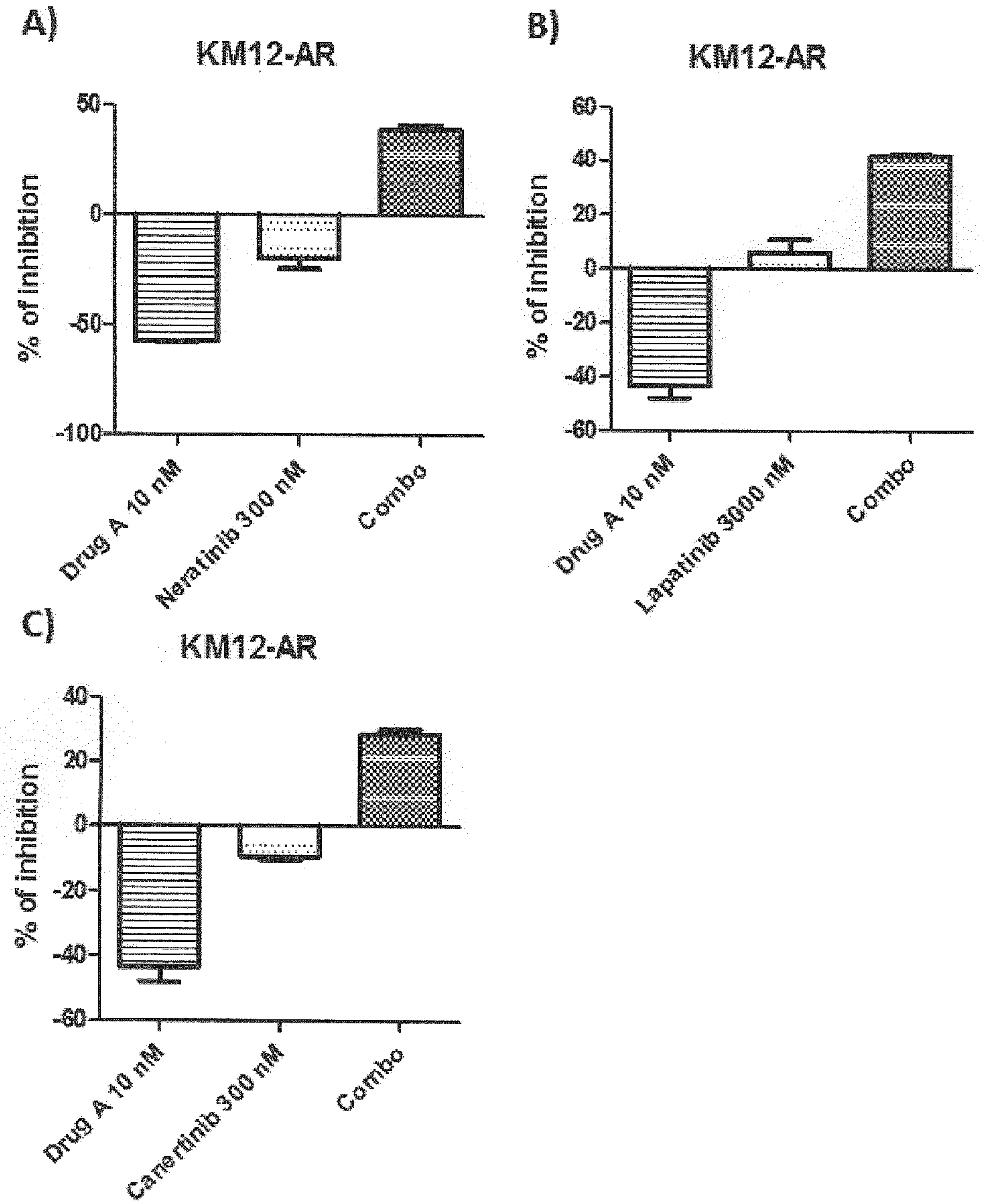
A)
KM12-AR
% of inhibition
50
0
-50
-100
Drug A 10 nM
Neratinib 300 nM
Combo
B)
KM12-AR
% of inhibition
60
40
20
0
-20
-40
-60
Drug A 10 nM
Lapatinib 3000 nM
Combo
C)
KM12-AR
% of inhibition
40
20
0
-20
-40
-60
Drug A 10 nM
Canertinib 300 nM
Combo

**INTERNATIONAL SEARCH REPORT**

International application No. PCT/JP2018/032880

A. CLASSIFICATION OF SUBJECT MATTER
Int.Cl. A61K31/444(2006.01)i, A61K31/4184(2006.01)i, A61K31/506(2006.01)i, A61K31/519(2006.01)i, A61K45/00(2006.01)i, A61P35/00(2006.01)i, A61P35/02(2006.01)i, A61P43/00(2006.01)i

According to International Patent Classification (IPC) or to both national classification and IPC

B. FIELDS SEARCHED

Minimum documentation searched (classification system followed by classification symbols)
Int.Cl. A61K31/00-33/44, A61K45/00, A61P1/00-43/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII), CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X<br>A | WO 2014/129431 A1 (ONO PHARMACEUTICAL CO., LTD.) 28 August 2014, claims & US 2016/0000783 A1, claims & EP 2960234 A1 & CN 104995172 A & KR 10-2015-0118148 A | 16<br>1-15, 17 |
| X<br>A | WO 2016/027754 A1 (ONO PHARMACEUTICAL CO., LTD.) 25 February 2016, claims & US 2017/0240527 A1, claims & EP 3184519 A1 & CN 106573913 A & KR 10-2017-0042591 A | 16<br>1-15, 17 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 November 2018 (28.11.2018) | 11 December 2018 (11.12.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2018/032880

C (Continuation). DOCUMENTS CONSIDERED TO BE RELEVANT

| Category* | Citation of document, with indication, where appropriate, of the relevant passages | Relevant to claim No. |
|---|---|---|
| A | WO 2013/161919 A1 (ONO PHARMACEUTICAL CO., LTD.) 31 October 2013, claims & US 2015/0111865 A1, claims & EP 2842955 A1 | 1-17 |
| A | DRILON, A. et al., "What hides behind the MASC: clinical response and acquired resistance to entrectinib after ETV6-NTRK3 identification in a mammary analogue secretory carcinoma (MASC)", Ann. oncol., May 2016, vol. 27, no. 5, pp. 920-926, ISSN 0923-7534 | 1-17 |
| A | RUSSO, M. et al., "Acquired Resistance to the IRK Inhibitor Entrectinib in Colorectal Cancer", Cancer Discov., January 2016, vol. 6, pp. 36-44, ISSN 2159-8290 | 1-17 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014129431 A
- WO 2016027754 A
- WO 2017106492 A
- US 2868691 A
- US 3095355 A


### Non-patent literature cited in the description


- *Annual Review of Biochemistry,* 2003, vol. 72, 609-642
- *Cancer Letters,* 2005, vol. 228, 143-153
- *Clinical Cancer Research,* 2009, vol. 15, 5962-5967
- *Proceedings of the National Academy of Sciences,* 2014, vol. 111, 10299-10304
- *Clinical Cancer Research,* 2001, vol. 7, 105-112
- *Carcinogenesis,* 2010, vol. 31, 1939-1947
- *Lung Cancer,* 2013, vol. 79, 205-214
- *Biochemical and Biophysical Research Communications,* 2013, vol. 441, 431-437
- *Nature Medicine,* 2013, vol. 19, 1469-1474
- *Cancer Discovery,* 2015, vol. 5, 25-34
- *Onco gene,* 2013, vol. 32, 3698-3710
- Iyakuhin no Kaihatsu (Development of Medicines). Bunshi Sekkei (Molecular Designs). Hirokawa Shoten Co, 1990, vol. 7, 163-198
- *Advances in enzyme regulation,* 1984, vol. 22, 27-55